# EUROPÄISCHE PATENTSCHRIFT

(11) **EP 1 263 387 B1**
(45) Veröffentlichungstag und Bekanntmachung des Hinweises auf die Patenterteilung: **15.09.2004**
(21) Anmeldenummer: 00989911.3
(22) Anmeldetag: 29.11.2000
(51) Int. Cl.: A61J 1/00, A61M 5/178, A61M 5/315

(54) **VERFAHREN ZUR REKONSTITUTION EINER INJEKTIONSFLÜSSIGKEIT, UND INJEKTIONSGERÄT ZUR DURCHFÜHRUNG EINES SOLCHEN VERFAHRENS**
METHOD FOR RECONSTITUTING AN INJECTION LIQUID AND AN INJECTION APPLIANCE FOR CARRYING OUT SUCH A METHOD
PROCEDE SERVANT A LA RECONSTITUTION D'UN LIQUIDE POUR INJECTION ET APPAREIL POUR INJECTION SERVANT A L'EXECUTION D'UN TEL PROCEDE

(30) Priorität: 16.02.2000 DE 10006935
(43) Veröffentlichungstag der Anmeldung: 11.12.2002
(73) Patentinhaber: Haselmeier S.à r.l., 1295 Mies (CH)
(72) Erfinder: POLZIN, Ulf, 70771 Leinfelden (DE)
(74) Vertreter: Raible, Hans, Dipl.-Ing.
(86) Internationale Anmeldenummer: PCT/EP2000/011916
(87) Internationale Veröffentlichungsnummer: WO 2001/060311

(56) Entgegenhaltungen:
- EP-A- 0 498 737
- WO-A-98/42394
- US-A- 5 472 022

## Beschreibung

Die Erfindung betrifft ein Verfahren zur Rekonstitution einer Injektionsflüssigkeit, und ein Injektionsgerät, das sich dafür eignet, eine Injektionsflüssigkeit vor der Injektion zu rekonstituieren.

Die Druckschrift WO-A-98/42 394 offenbart ein Injectionsgerät gemäß dem Oberbegriff des Anspruchs 1.

Insbesondere Proteine haben in gelöster Form keine lange Lebensdauer und müssen deshalb, vom Lösungsmittel getrennt und meist in lyophilisierter Form, gelagert werden. Erst unmittelbar vor der ersten Injektion wird der Wirkstoff in dem Lösungsmittel gelöst.

Es ist deshalb eine Aufgabe der Erfindung, ein neues Verfahren zur Rekonstitution einer Injektionsflüssigkeit, und ein Injektionsgerät zur Durchführung eines solchen Verfahrens, bereitzustellen.

Nach der Erfindung wird diese Aufgabe gelöst durch den Gegenstand des Patentanspruchs 1. Ein solches Gerät ist einfach und sinnfällig zu bedienen, denn der Patient versteht und sieht, was bei der Rekonstitution vor sich geht, und nach der Rekonstitution ist das Gerät bereit für eine Injektion, ohne dass der Patient hierfür noch weitere komplizierte Schritte ausführen muss.

Ein erfindungsgemäßes Verfahren ist Gegenstand des Anspruchs 26. Ein solches Verfahren ist einfach und kann auch von Laien durchgeführt werden, gewöhnlich nach entsprechender Unterrichtung in einem Krankenhaus oder bei einem Facharzt.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel, sowie aus den Unteransprüchen. Es zeigt:
- Fig. 1: eine Außenansicht eines erfindungsgemäßen Injektionsgeräts im Zustand, wie es die Fabrik verlässt, also vor der Rekonstitution des Lyophilisates,
- Fig. 2: eine Außenansicht des Injektionsgeräts im Zustand nach der Vorbereitung für Injektionen, also nach der Rekonstitution des Lyophilisates, und nach dem Einstellen einer Injektionsdosis von sechs Einheiten,
- Fig. 3: eine vergrößerte Darstellung des Ausschnittes III der Fig. 2,
- Fig. 4: einen Längsschnitt durch das Injektionsgerät der Fig. 1, zusammen mit einer Verlängerungsstange 126, die bei der Rekonstitution benutzt wird,
- Fig. 5: einen Längsschnitt analog Fig. 4, aber senkrecht zur Ebene der Fig. 4,
- Fig. 6: eine vergrößerte Darstellung des mittleren Teils der Fig. 5,
- Fig. 7: eine Ausschnittsvergrößerung der in Fig. 6 mit VII bezeichneten Einzelheit,
- Fig. 8: einen Schnitt, gesehen längs der Linie VIII-VIII der Fig. 5,
- Fig. 9: einen Schnitt, gesehen längs der Linie IX-IX der Fig. 5,
- Fig. 10: eine raumbildliche Darstellung eines beim Ausführungsbeispiel verwendeten Stellglieds, das zur Vorbereitung einer Injektion im Gehäuse des Injektionsgeräts verschoben wird,
- Fig. 11: einen teilweisen Längsschnitt durch eine Zahnstange, gesehen längs der Linie XI-XI der Fig. 13,
- Fig. 12: einen teilweisen Längsschnitt analog Fig. 11, aber gesehen längs der Linie XII-XII der Fig. 13; der obere Teil ist ungeschnitten dargestellt,
- Fig. 13: einen Schnitt, gesehen längs der Linie XIII-XIII der Fig. 12,
- Fig. 14: einen Längsschnitt durch ein bevorzugtes Verbindungsteil nach der Erfindung,
- Fig. 15: einen Schnitt durch das Verbindungsteil der Fig. 14, mit dem ein Behälter mit einem lyophilisierten Arzneimittel verbunden ist,
- Fig. 16: einen Längsschnitt analog Fig. 4 durch ein erfindungsgemäßes Injektionsgerät, an dem eine Anordnung gemäß Fig. 15 befestigt worden ist,
- Fig. 17: einen Längsschnitt durch die Anordnung der Fig. 16, aber orthogonal zur Schnittebene der Fig. 16,
- Fig. 18: eine vergrößerte Darstellung des Ausschnitts XIIX der Fig. 17,
- Fig. 19: eine Darstellung analog Fig. 16, aber nach Befestigen der in Fig. 4 dargestellten Verlängerungsstange 126,
- Fig. 20: eine vergrößerte Darstellung des in Fig. 19 mit XX bezeichneten Ausschnitts,
- Fig. 21: eine vergrößerte Darstellung analog Fig. 20, aber um 90° gegenüber der Schnittebene der Fig. 20 verdreht,
- Fig. 22: eine Darstellung analog Fig. 19, aber in dem Zustand, bei dem die Verlängerungsstange 126 in Richtung eines Pfeiles 242 voll in das Injektionsgerät 40 hineingeschoben worden ist, um einen großen Teil der Flüssigkeit 49 aus dem Injektionsgerät in den daran befestigten Behälter 224 zu pressen,
- Fig. 23: eine Darstellung analog Fig. 22, aber in einer zu Fig. 22 um 90° verdrehten Schnittebene,
- Fig. 24: eine vergrößerte Darstellung des in Fig. 23 mit XXIV bezeichneten Ausschnitts, zur Erläuterung der Wirkungsweise eines Signalglieds 131,
- Fig. 25: eine Darstellung analog Fig. 23, aber nach Zurücksaugen der rekonstituierten Injektionsflüssigkeit 226' in die Kartusche 50 des Injektionsgeräts 40 durch Ziehen der Stange 126 in Richtung eines Pfeiles 260,
- Fig. 26: eine vergrößerte Darstellung des in Fig. 25 mit XXVI bezeichneten Ausschnitts,
- Fig. 27: eine Darstellung analog Fig. 26, aber in einer zu Fig. 26 um 90° verdrehten Schnittebene,
- Fig. 28: einen Schnitt analog Fig. 16, aber nach der Rekonstitution und nach dem Entfernen des leeren Behälters 224 und seines Adapters 210,
- Fig. 29: eine Darstellung analog Fig. 28, aber nach Befestigen einer Injektionsnadel 268, und nach Einstellen der gewünschten Injektionsdosis,
- Fig. 30: eine Darstellung analog Fig. 29, welche den ersten Teil des Spannvorgangs zeigt, mit dem das Injektionsgerät für eine anschließende Injektion vorbereitet wird,
- Fig. 31: eine Schnittdarstellung analog Fig. 30, aber in einer relativ zu Fig. 30 um 90° verdrehten Schnittebene,
- Fig. 32: eine vergrößerte Darstellung des in Fig. 31 mit XXXII bezeichneten Ausschnitts,
- Fig. 33: eine Darstellung analog Fig. 30, welche den Zustand des Injektionsgeräts nach dem Abschluss des Spannvorgangs zeigt; das Gerät ist jetzt für eine Injektion bereit,
- Fig. 34: eine Schnittdarstellung analog Fig. 33, aber in einer zu Fig. 33 orthogonalen Schnittebene,
- Fig. 35: eine vergrößerte Darstellung des in Fig. 34 mit XXXV bezeichneten Ausschnitts,
- Fig. 36: einen Längsschnitt analog Fig. 29, aber nach Durchführung einer Injektion; auf die gegenüber Fig. 29 geänderte Stellung der Zahnstange 92 wird hingewiesen,
- Fig. 37: eine Schnittdarstellung analog Fig. 36, aber in einer gegenüber Fig. 36 um 90° verdrehten Schnittebene,
- Fig. 38: eine raumbildliche Darstellung eines beim Ausführungsbeispiel verwendeten Signalteils zur Anzeige der Rekonstitution,
- Fig. 39: einen Schnitt durch das Signalteil, gesehen längs der Linie XXXIX-XXXIX der Fig. 40,
- Fig. 40: eine Draufsicht auf das Signalteil, gesehen in Richtung des Pfeiles XXXX der Fig. 39,
- Fig. 41: einen Schnitt durch das Signalteil, gesehen längs der Linie XXXXI-XXXXI der Fig. 42, und
- Fig. 42: eine Draufsicht, gesehen in Richtung des Pfeiles XXXXII der Fig. 41.

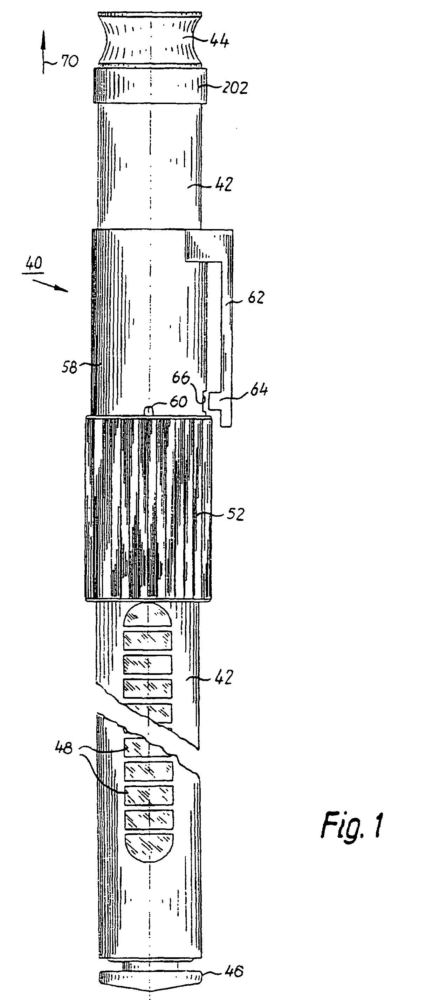

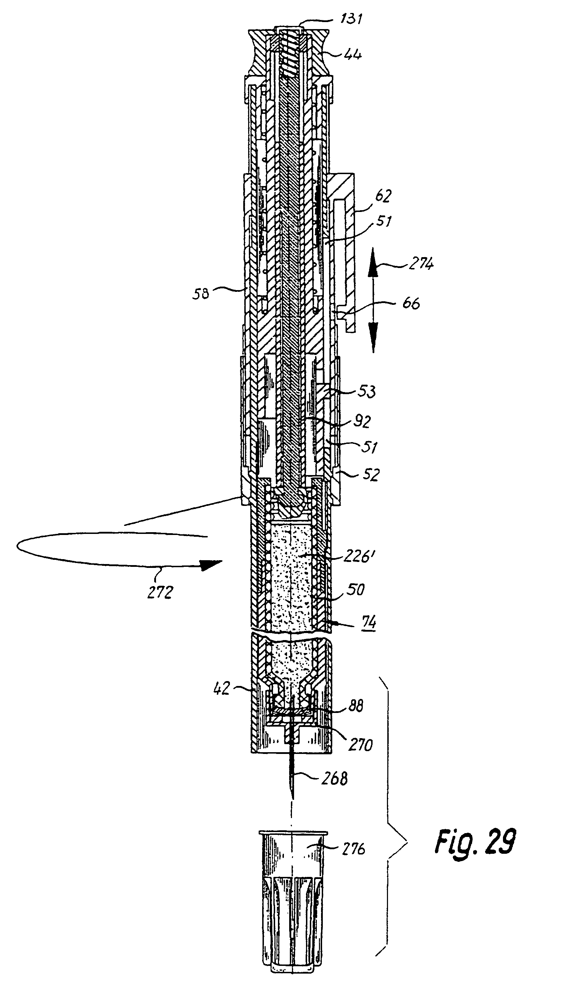

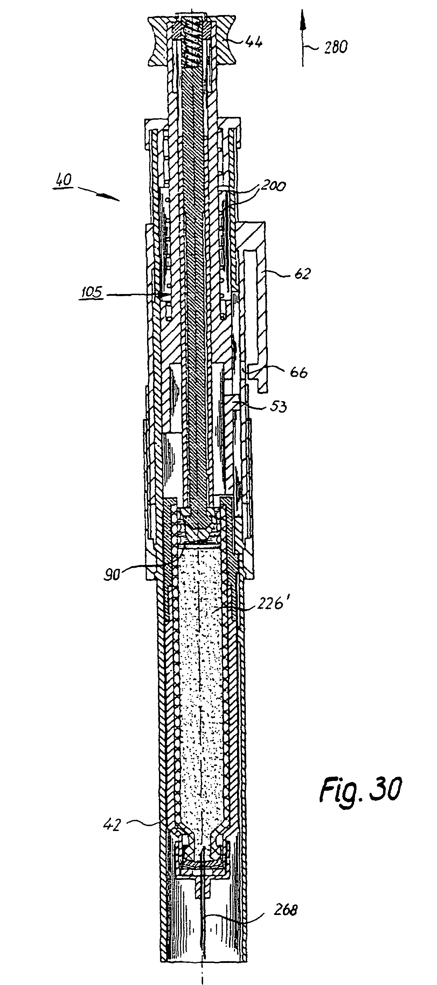

**Fig. 1** zeigt in vergrößertem Maßstab ein Injektionsgerät 40 in dem Zustand, wie es die Fabrik des Herstellers verlässt. Es hat das Aussehen eines überdimensionierten Füllfederhalters und hat ein längliches, rohrartiges Gehäuse 42. Am distalen Ende, also in Fig. 1 oben, befindet sich ein Betätigungsknopf 44, der zum Spannen des Geräts vor einer Injektion dient, vgl. die Fig. 30 bis 35, und unten, also am proximalen Ende, wo eine Abdeckkappe 46 dargestellt ist, wird vor Gebrauch eine Nadel befestigt, vgl. Fig. 29.

Wie in der Medizin und Anatomie üblich, werden die Begriffe distal, medial und proximal verwendet wie folgt:
- Proximal:: Die dem Patienten zugewandte Seite, also die Seite mit der Nadel (in Fig. 1 unten).
- Distal:: Die vom Patienten abgewandte Seite, also in Fig. 1 die Seite mit dem Betätigungsknopf 44.
- Medial:: Zur Mittelachse des Geräts 40 gerichtet; nahe bei der Mittelachse.

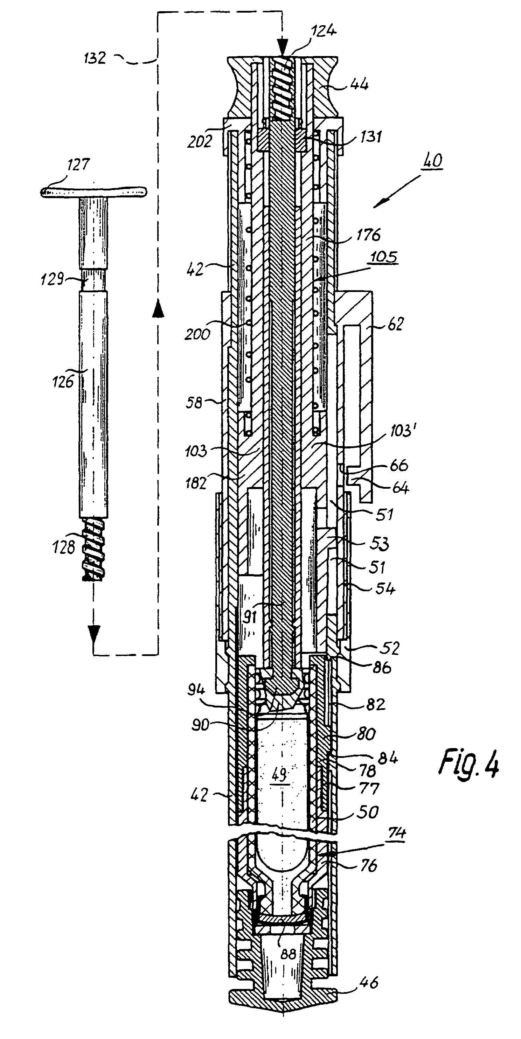

Im proximalen Teil des Gehäuses 42 befindet sich ein Fenster 48, durch das der Patient sehen kann, wie viel Flüssigkeit 49 in einer dortigen Kartusche 50 (Fig. 4) vorhanden ist. Im distalen Teil des Gehäuses 42 befindet sich eine längliche Öffnung 51 (Fig. 4), welche zur axialen Führung eines federnden Rastglieds 53 dient.

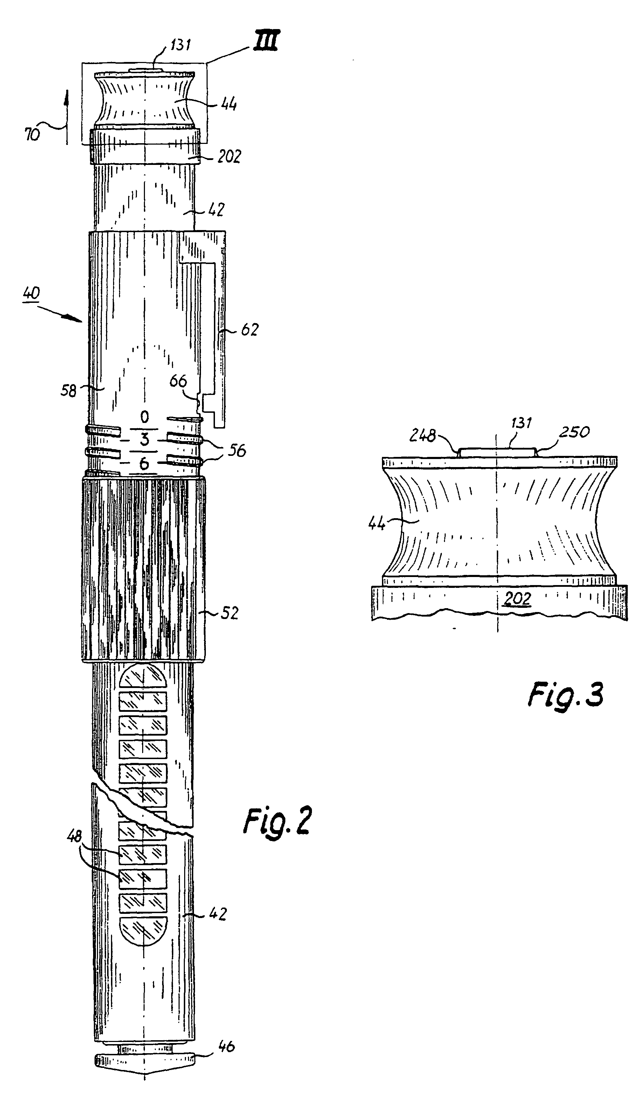

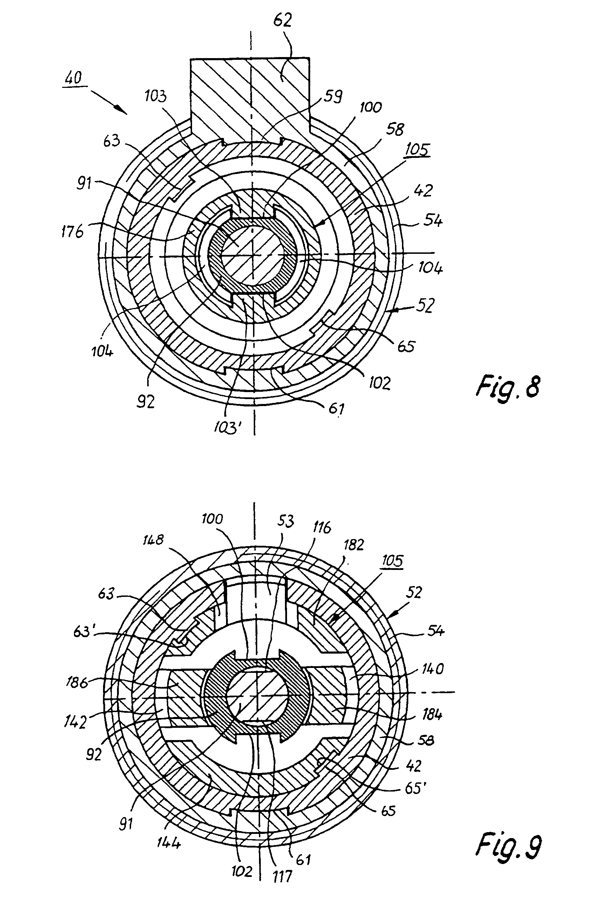

Auf dem mittleren Teil des Gehäuses 42 ist ein Dosierrad 52 drehbar, aber axial unverschiebbar, angeordnet. Es hat ein Innengewinde 54 (Fig. 4), das in ein Außengewinde (Steilgewinde) 56 (Fig.2) eines Dosiseinstellteils 58 eingreift, welches in Längsnuten 59, 61 des Gehäuses 42 axial verschiebbar, aber unverdrehbar, geführt ist, vgl. Fig. 8. Wird das Dosierrad 52 gedreht, so wird das Dosiseinstellteil 58 axial verschoben, wie ein Vergleich der Fig. 1 und 2 zeigt, d.h. Fig. 1 zeigt die Dosiseinstellung "0", erkennbar an der bei 60 dargestellten "0", und Fig. 2 zeigt die Dosiseinstellung "6". Man kann also durch Verdrehen des Dosierrades 52 die Injektionsdosis einstellen. Wird die eingestellte Injektionsdosis nicht verändert, so wird bei jeder Injektion dieselbe Dosis injiziert. Man bezeichnet das als "Steady Dose Injection". Die Dosierung kann aber auf Wunsch vor jeder Injektion verändert werden, sofern nicht das Dosierrad 52 immobilisiert worden ist.

Am Dosiseinstellteil 58 ist ein federnder Clip 62 vorgesehen, der zur Auslösung einer Injektion dient. An seinem freien Ende hat er einen zum Gerät 40 ragenden, medialen Vorsprung 64, der einer Rastöffnung 66 des Dosiseinstellteils 58 gegenüberliegt. Ein Vergleich der Fig. 1 und 2 zeigt, wie diese Öffnung 66, zusammen mit dem Clip 62, bei Erhöhung der Dosis in distaler Richtung verschoben wird. Die Rastöffnung 66 fluchtet mit der länglichen Öffnung 51 des Gehäuses 42.

Fig. 1 zeigt am distalen Ende, also oben, einen Pfeil 70, um anzudeuten, dass der Betätigungsknopf 44 in Richtung dieses Pfeiles 70 in distaler Richtung verschoben werden kann. Der Knopf 44 ist nicht verdrehbar, wie sich aus der nachfolgenden Beschreibung ergibt.

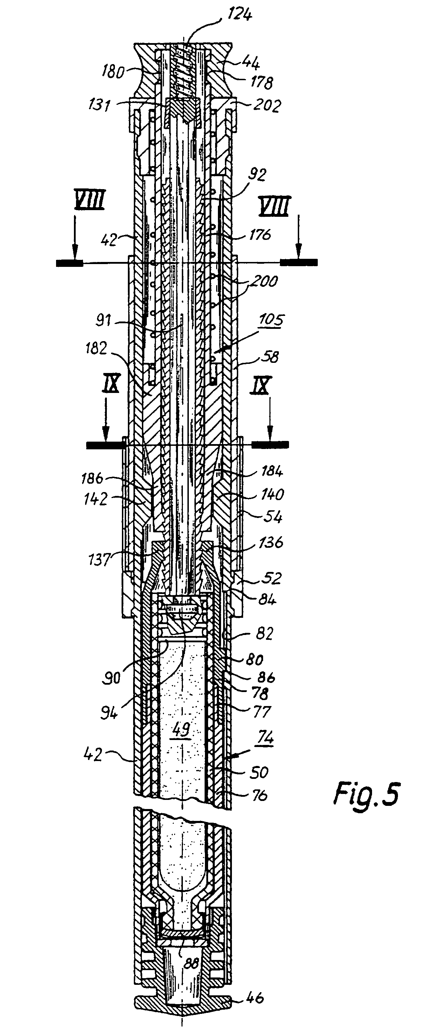

**Fig. 4** ist ein Längsschnitt durch das Injektionsgerät 40 der Fig. 1, und Fig. 5 ein analoger Längsschnitt, aber in einer zu Fig. 4 senkrechten Ebene.

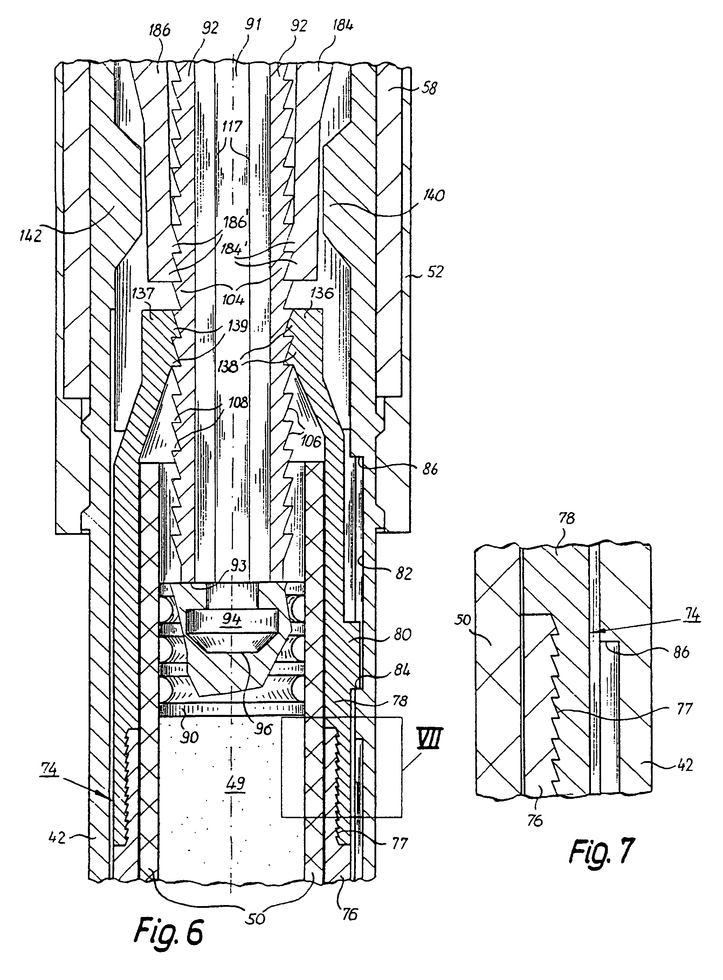

Im proximalen Teil des Gehäuses 42 befindet sich ein transparenter Kartuschenhalter 74, der aus einem proximalen Teil 76 und einem distalen Teil 78 zusammengesetzt ist und in seinem Inneren die Kartusche 50 festhält. Die Teile 76, 78 sind miteinander durch eine Mikrorastung 77 verbunden, die in Fig. 6 und 7 in stärkerer Vergrößerung dargestellt ist. Sie gestattet eine einfache und dabei hochpräzise Einstellung der Länge des Kartuschenhalters 74 zur Anpassung an die Kartusche 50 und deren Füllmenge. Diese Füllmenge kann infolge von Ungenauigkeiten bei der Fabrikation und beim Füllen der Kartuschen variieren. Fig. 6 und 7 zeigen die kürzest mögliche Länge des Kartuschenhalters 74.

Das Teil 78 hat radiale Vorsprünge 80, die in entsprechenden Längsnuten 82 des Gehäuses 42 geführt und dort zwischen zwei Anschlägen 84, 86 verschiebbar sind. Dadurch ist der Kartuschenhalter 74 relativ zum Gehäuse 42 nicht verdrehbar und nur in begrenztem Maß axial verschiebbar. Fig. 6 zeigt den Vorsprung 80 in stärkerer Vergrößerung.

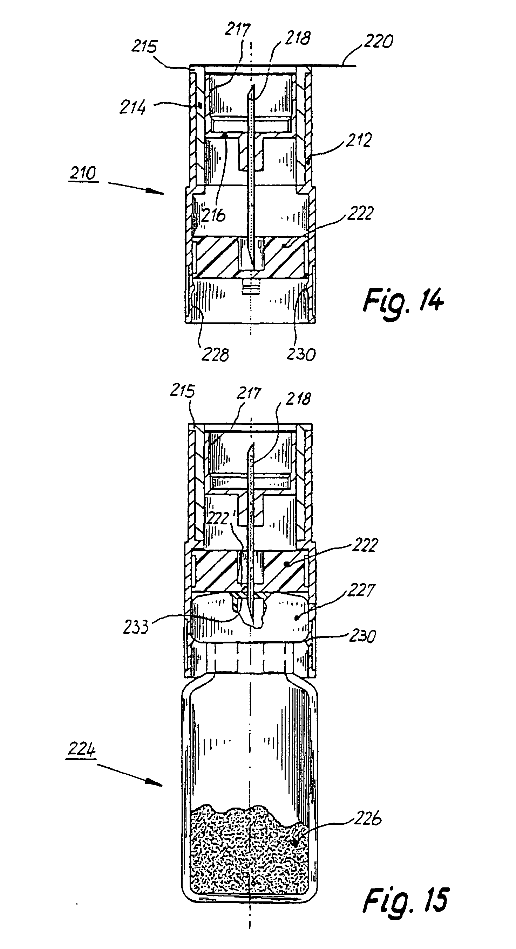

Im Zustand nach Fig. 1 und 4 bis 6 ist die Flüssigkeit 49 in der Kartusche 50 nur ein Lösungsmittel, z.B. Kochsalzlösung. Diesem Lösungsmittel muss noch ein Wirkstoff beigefügt werden, der gewöhnlich als Lyophilisat in einem sterilen Behälter vorliegt, vgl. den Behälter 224 in Fig. 15 mit dem Lyophilisat 226. Wie dies geschieht, wird nachfolgend im einzelnen beschrieben. Man nennt das die Rekonstitution der Flüssigkeit 49, oder anders gesagt: Deren Umwandlung in ein Arzneimittel, das anschließend vom Patienten injiziert werden kann. Hierzu muss das Injektionsgerät entsprechend "präpariert" werden, ehe es vom Patienten in Benutzung genommen werden kann.

Der Grund für die getrennte Lagerung von Lösungsmittel und Wirkstoff ist, dass die rekonstituierte Flüssigkeit nur eine begrenzte Haltbarkeitsdauer hat, z.B. 2 bis 3 Wochen. Die Rekonstitution wird meist von Fachpersonal vorgenommen (Krankenschwester, Arzt), kann aber auch vom Patienten selbst vorgenommen werden, wenn dieser entsprechend unterrichtet worden ist. Ein wesentlicher Vorteil eines Injektionsgeräts nach der vorliegenden Erfindung ist die einfache und leicht verständliche Rekonstitution, die auch von einem Laien problemlos durchgeführt werden kann.

In der üblichen Weise hat die Kartusche 50 an ihrem proximalen Ende eine Gummimembran 88, die von einer Nadel durchstochen werden kann, vgl. Fig. 29. An ihrem distalen Ende hat sie einen Gummikolben 90, an welchem das proximale Ende eines Betätigungsgliedes 91 befestigt ist, das in Fig. 6 in einer Ausschnittsvergrößerung teilweise dargestellt ist. Hierzu ist das proximale Ende des Betätigungsglieds 91 mit einem pilzartigen Vorsprung 94 in eine hierzu komplementäre Ausnehmung 96 des Kolbens 90 eingeknöpft, vgl. Fig. 6. Alternativ kann der Kolben 90 auch über eine Gewindeverbindung mit dem Betätigungsglied 91 verbunden werden.

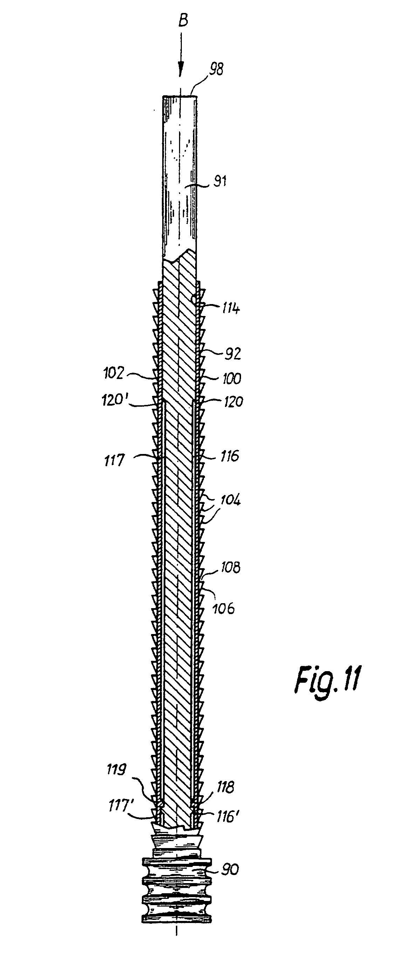

Das Betätigungsglied 91 ist bei dieser Ausführungsform axial verschiebbar und unverdrehbar im Inneren einer als Stößel dienenden Zahnstange 92 (Fig. 11 bis 13) geführt, die mit ihrem proximalen Ende 93 gegen die distale Seite des Kolbens 90 anliegt, jedoch mit diesem nicht fest verbunden ist. Die Zahnstange 92 dient zur Durchführung von Injektionen, wobei sie den Kolben 90 nur in proximaler Richtung verschieben kann, nicht aber in distaler Richtung.

Das Betätigungsglied 91 dient zur Durchführung einer Rekonstitution, weshalb es den Kolben 90 sowohl in proximaler wie in distaler Richtung verschieben kann. Bei der distalen Verschiebung des Kolbens 90 dient das proximale Ende 93 der Zahnstange 92 als Anschlag, vgl. Fig. 11.

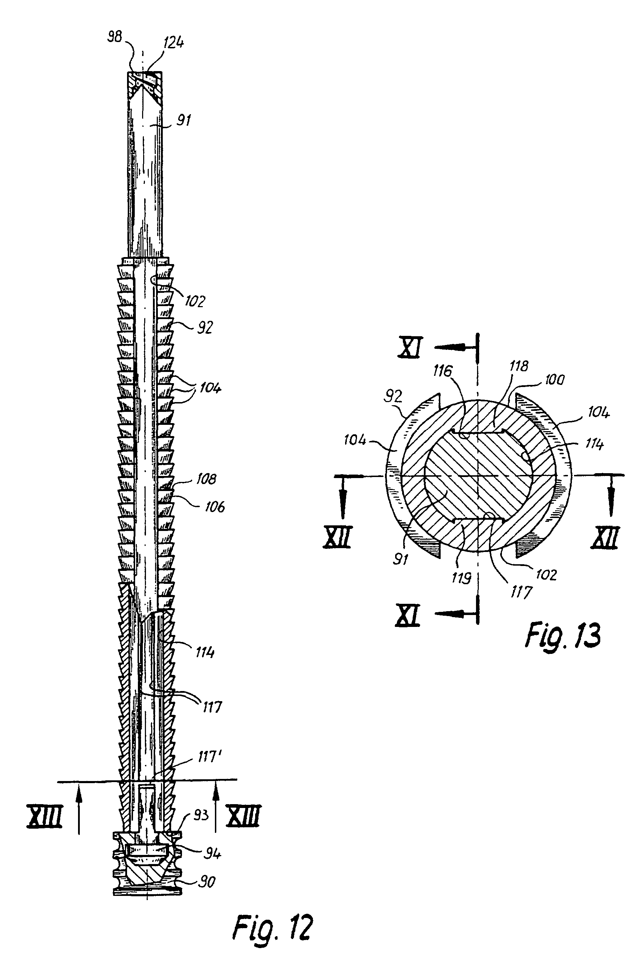

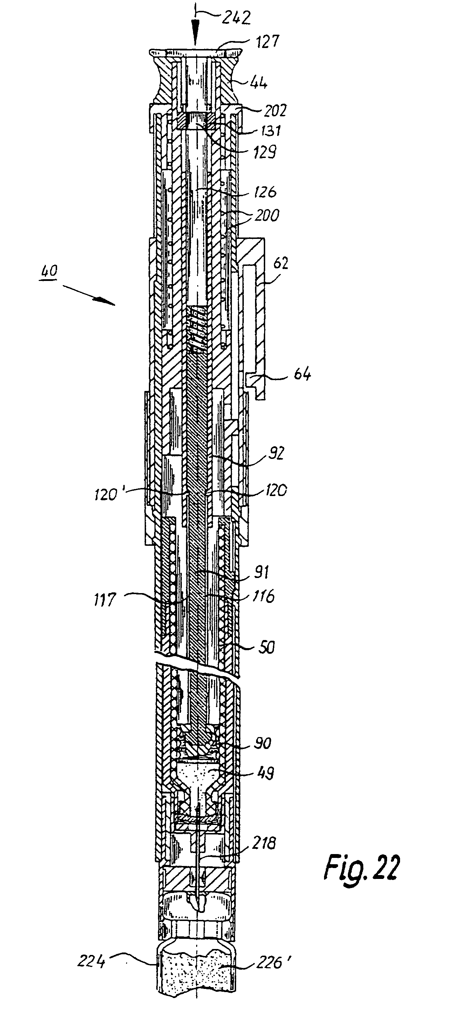

Fig. 11 zeigt einen teilweisen Längsschnitt durch das Betätigungsglied 91 und die Zahnstange 92, gesehen in Richtung der Pfeile XI-XI der Fig. 13. Das Betätigungsglied 91 hat in seinem distalen Bereich einen kreiszylindrischen Außenumfang, und es hat auf einem Teil seiner Längserstreckung zwei gegenüberliegende Längsnuten 116, 117, die durch Vorsprünge 118, 119 auf der Innenseite einer im wesentlichen zylindrischen Ausnehmung 114 der Zahnstange 92 geführt sind. Die Längsnuten 116, 117 erstrecken sich in distaler Richtung bis zu distalen Nutenden 120, 120' (Fig. 22), und wenn letztere bei der Rekonstitution gegen die Anschläge 118, 119 stoßen, begrenzt dies die Bewegung des Betätigungsglieds 91 in proximaler Richtung, vgl. Fig. 22. Umgekehrt befinden sich in den Längsnuten 116, 117 Vorsprünge 116', 117', welche die Bewegung des Betätigungsglieds 91 in distaler Richtung begrenzen, wenn sie gegen die Anschläge 118, 119 stoßen, wie das in Fig. 11 dargestellt ist. Eine solche Bewegung in distaler Richtung findet am Ende einer Rekonstitution statt.

Wie in den Fig. 11 bis 13 dargestellt, ist der Kolben 90 auf dem pilzförmigen proximalen Ende 94 des Betätigungsglieds 91 so befestigt, dass er den Bewegungen des Betätigungsglieds 91 in proximaler und distaler Richtung folgt.

Gemäß Fig. 13 hat die Zahnstange 92 hat einen im wesentlichen kreisrunden Querschnitt mit zwei gegenüberliegenden Längsnuten 100, 102 zur axialen Längsführung. Diese Nuten sind geführt in nach innen ragenden Vorsprüngen 103, 103' (Fig. 4, 8) eines Stellglieds 105, das seinerseits durch Vorsprünge 63, 65 (Fig. 8, 9) und Längsnuten 63', 65' (Fig. 8, 9, 10) im Inneren des Gehäuses 42 unverdrehbar geführt ist, so dass die Zahnstange 92 relativ zum Gehäuse 42 nicht verdreh-, sondern nur axial verschiebbar ist.

Die Fig 11 und 12 zeigen in vergrößertem Maßstab die Zähne 104 der Zahnstange 92. Diese haben die für eine Zahnstange charakteristische Sägezahnform, d.h. der proximale Teil 106 eines Zahnes 104 hat eine flache Steigung, der distale Teil 108 dagegen eine große Steigung.

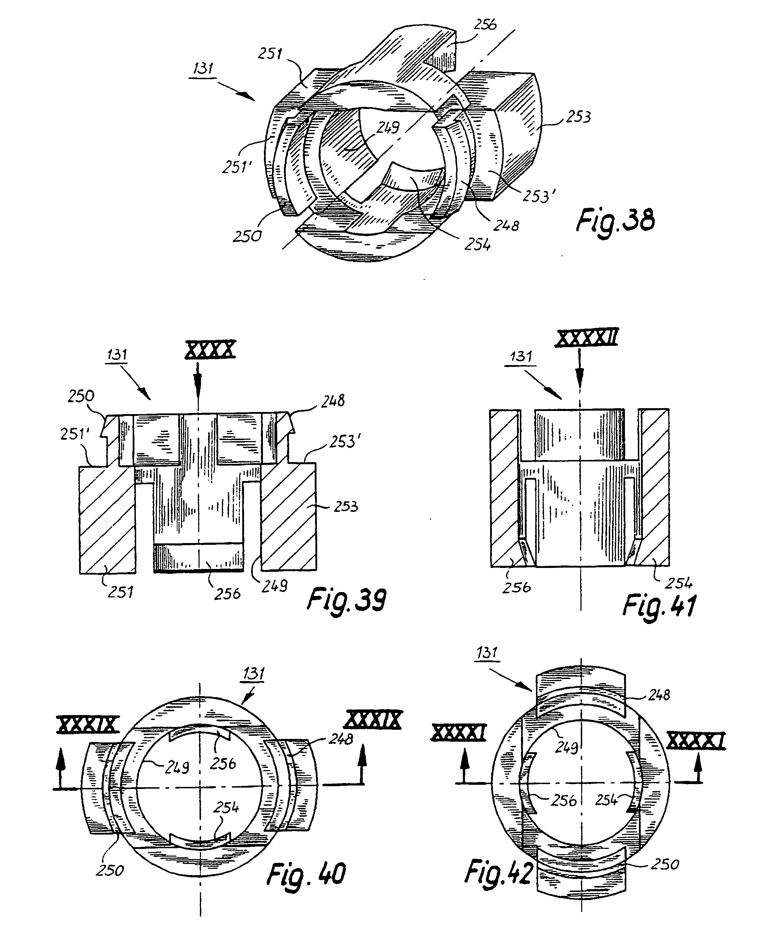

Das distale Ende des Betätigungsglieds 91 hat ein Innengewinde 124 (Fig. 4 und 12), in das die Verlängerungsstange 126 mit ihrem Außengewinde 128 eingeschraubt werden kann, wie in Fig. 4 durch eine gestrichelte Linie 132 angedeutet, um das Betätigungsglied 91 während der Rekonstitution entsprechend zu verlängern. Die Verlängerungsstange 126 hat an ihrem distalen Ende einen Betätigungsknopf 127; das Außengewinde 128 befindet sich am proximalen Ende. Ferner hat sie im Bereich ihres distalen Endes eine Ringnut 129, welche mit einem Signalglied 131 (Fig. 38 bis 42) zusammenwirkt, das auf dem Betätigungsglied 91 axial verschiebbar angeordnet ist. Das Signalglied 131 wird bei einer Rekonstitution aktiviert und signalisiert nach Abschluss der Rekonstitution, dass das Injektionsgerät 40 rekonstituierte Injektionsflüssigkeit enthält und für eine Injektion bereitsteht.

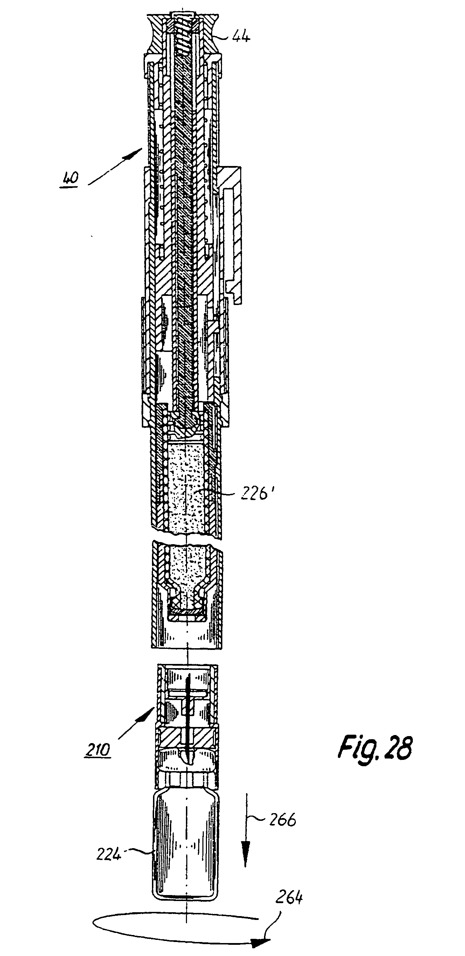

Die **Fig. 38 bis 42** zeigen Einzelheiten des Signalglieds 131. Aus der - stark vergrößerten - raumbildlichen Darstellung der Fig. 38 ist sein Aufbau gut erkennbar. Es hat im Prinzip die Struktur eines Rings, dessen Innenausnehmung 249 auf der Außenseite des Betätigungsglieds 91 bzw. der Verlängerungsstange 126 gleitet. Von diesem Ring erstrecken sich in distaler Richtung zwei Rasthaken 248, 250, und in proximaler Richtung zwei federnden Abschnitte 254, 256. Außerdem hat das Signalglied 131 zwei radial nach außen ragende Abschnitte 251 und 253, die nach der Rekonstitution mit ihren distalen Stirnflächen 251' bzw. 253' in der Lage gemäß Fig. 27 gegen den Drehknopf 44 zur Anlage kommen, wobei die Rasthaken 248, 250 im Drehknopf 44 einrasten, wie das die Fig. 2 und 3 zeigen. Diese Stellung des Signalglieds 131 zeigt an, dass das Gerät rekonstituierte Injektionsflüssigkeit enthält.

Wie die **Fig. 5 und 6** zeigen, befinden sich am distalen Endbereich des Kartuschenhalters 74 zwei federnde Teile 136, 137, die man auch als Spannbacken bezeichnen kann und die auf ihrer medialen Seite mit Rastzähnen 138 bzw. 139 zum Eingriff in die Zähne 104 der Zahnstange 92 versehen sind. Die federnden Teile 136, 137 liegen in der Lage gemäß Fig. 5 und 6 federnd gegen die Zahnstange 92 an, haben also eine Vorspannung in medialer Richtung, ermöglichen aber eine Verschiebung der Zahnstange 92 in proximaler Richtung, weil sie entgegen der medialen Richtung ausfedern können.

Die Schnittdarstellung der **Fig. 8** zeigt, wie das Dosiseinstellteil 58, das den Auslöseclip 62 trägt, in zwei Längsnuten 59, 61 des Gehäuses 42 längsverschiebbar geführt ist.

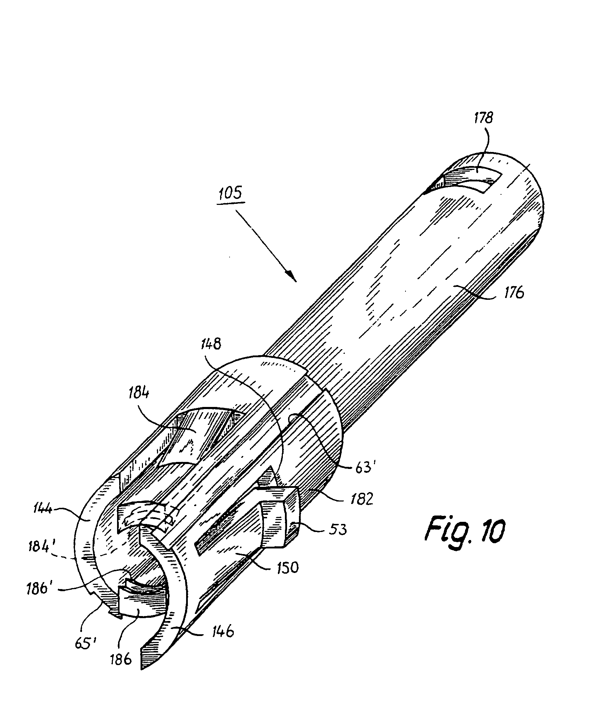

Auf seiner Innenseite hat das Gehäuse 42 zwei in Längsrichtung verlaufende Vorsprünge 63, 65. Diese dienen dazu, das Stellglied 105, das in Fig. 10 raumbildlich dargestellt ist, in Längsrichtung zu führen und gegen Verdrehung zu sichern. Das Stellglied 105 besteht, wie in Fig. 10 dargestellt, aus einem rohrförmigen Teil 176 kleineren Durchmessers und einem Teil 182 größeren Durchmessers. Letzteres hat Längsnuten 63', 65', und diese werden von den Vorsprüngen 63, 65 des Gehäuses 42 geführt.

An seinem distalen Endbereich hat das Rohr 176 zwei Rastausnehmungen 178, die gemäß Fig. 5 zur Befestigung der radialen Vorsprünge 180 des Betätigungsknopfes 144 dienen. Das Rohr 176 hat auf seiner Innenseite zwei axial verlaufende Vorsprünge 103, 103' (Fig. 8), welche in die Längsnuten 100, 102 der Zahnstange 92 eingreifen und diese gegen Verdrehung sichern.

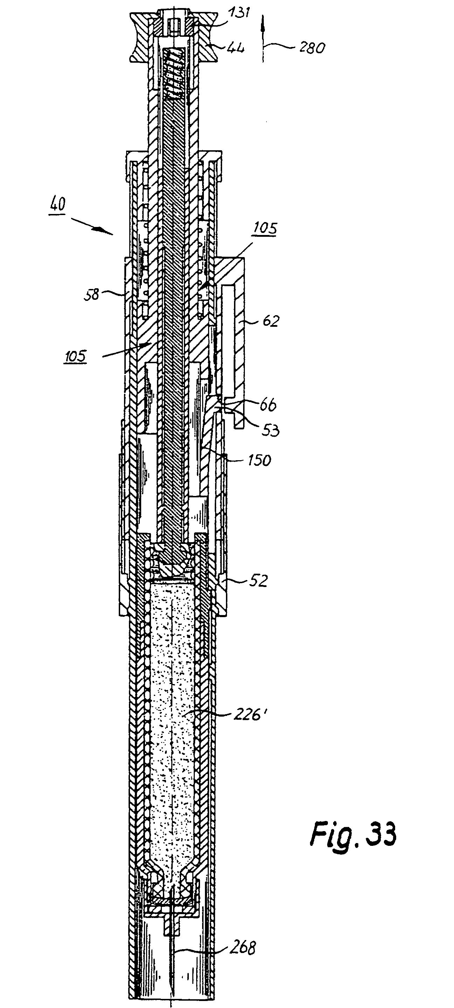

Der Abschnitt 182 des Stellglieds 105, der in raumbildlicher Form in Fig. 10 dargestellt ist, hat eine kürzere Halbschale 144 und eine längere Halbschale 146, die sich vom Rohr 176 weg in proximaler Richtung erstrecken. In der Halbschale 146 befindet sich eine U-förmige Ausnehmung 148, welche eine federnde Zunge 150 definiert, an deren distalem, freiem Ende sich das nach außen ragende Rastglied 53 befindet, das vor einer Injektion in die Rastöffnung 66 des Dosiseinstellteils 58 zum Einrasten gebracht wird, vgl. Fig. 33. In der Stellung gemäß Fig. 5 und 9 liegt es mit federnder Vorspannung gegen die Innenseite des Dosiseinstellteils 58 an und ist dadurch radial nach innen verschoben. In der Stellung gemäß Fig. 10 ist die federnde Zunge 150 nach außen gefedert. Dies entspricht der Stellung, die in Fig. 33 dargestellt ist.

Ferner hat das Teil 182 zwei federnde Spannbacken 184, 186, die zwischen den Halbschalen 144, 146 liegen und auf ihrer der Zahnstange 92 zugewandten Seite mit Rastzähnen 184', 186' versehen sind. Diese sind etwa komplementär zu den Zähnen 104 der Zahnstange 92 ausgebildet und greifen mit elastischer Vorspannung in diese Zähne 104 ein.

Eine distale Schulter des Teils 182 dient als Widerlager für das proximale Ende einer Injektionsfeder 200 (in Fig. 8 nicht dargestellt), deren distales Ende gegen eine Abschlusskappe 202 anliegt, die in der in Fig. 21 dargestellten Weise in das distale Ende des Gehäuses 42 eingerastet ist und eine Führung für das Rohr 176 bildet.

Für die Rekonstitution wird ein Adapter 210 verwendet, der in Fig. 14 und 15 dargestellt ist. Er hat ein etwa zylindrisches Gehäuse 212, in welchem ein Kanülenhalter 214 befestigt ist, z.B. durch Einpressen, Kleben oder Verschweißen. Dieser hat einen oberen Flansch 215, der radial über den oberen Rand des Gehäuses 212 übersteht. Im Kanülenhalter 214 ist ein Standard-Kanülenträger 216 mit seiner Kanüle 218 in geeigneter Weise befestigt. Der Kanülenhalter 214 ist oben mit einer Peelfolie 220 steril verschlossen, welche auf dem Flansch 215 lösbar befestigt ist. Im Kanülenträger 216 ist ein Innengewinde 217 zur Befestigung am Injektionsgerät 40 vorgesehen.

Im unteren Teil des Gehäuses 212 befindet sich ein verschiebbarer Gummikolben 222, der im unbenutzten Zustand das untere Ende der Kanüle 218 steril abdeckt, während das obere Ende von der Peelfolie 220 abgedeckt ist. Dies ermöglicht eine sterile Lagerung der Kanüle 218.

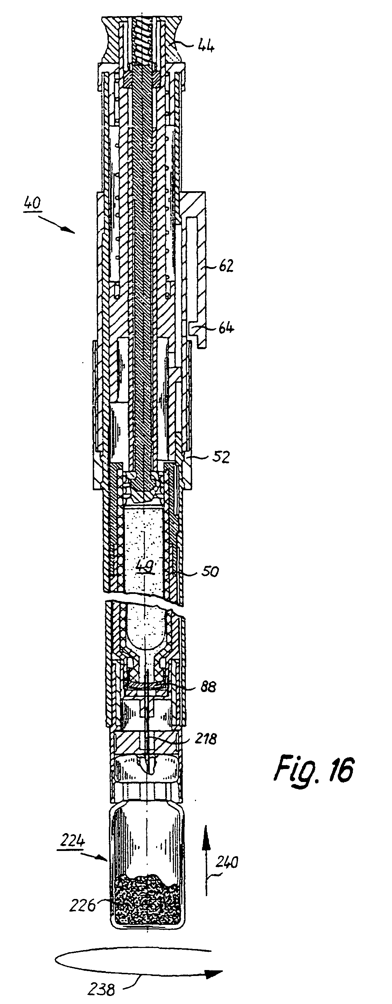

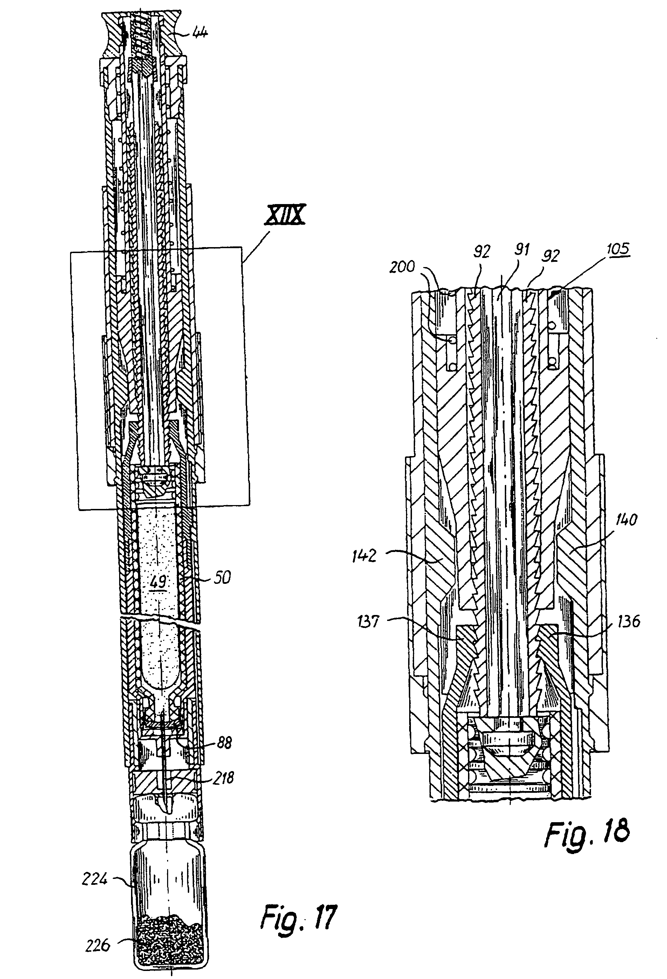

Wie Fig. 15 zeigt, wird ein Behälter 224, der ein Lyophilisat 226 enthält, mit seinem Deckel 227 in die untere Ausnehmung 228 des Gehäuses 212 gepresst und verrastet dort an einem Rastvorsprung 230. Dabei verschiebt er den Gummikolben 222 nach oben, und dieser wird, wie dargestellt, an einer dünnen Mittelpartie 222' vom unteren Ende der Kanüle 218 durchstochen, welche anschließend eine Gummimembran 233 im Deckel 227 durchsticht. Anschließend wird die Peelfolie 220 abgezogen, und die Anordnung nach Fig. 15 kann, genauso wie eine Injektionsnadel, mittels des Innengewindes 217 an das Injektionsgerät 40 angeschraubt werden, vgl. Fig. 16 und 17. Dabei durchdringt das in Fig. 14 und 15 obere Ende der Kanüle 218 die Gummimembran 88 der Kartusche 50 und bildet eine Verbindung von dieser zum Behälter 224, so dass die Flüssigkeit 49 aus der Kartusche 50 über die Kanüle 218 in den Behälter 224 fließen kann.

**Fig. 16** zeigt, wie die Anordnung gemäß Fig. 15 am proximalen Ende des Injektionsgeräts 40 angeschraubt wird, symbolisiert durch einen Drehpfeil 238 und einen Pfeil 240. Die Nadel 218 durchsticht dabei mit ihrem distalen Ende die Gummimembran 88 der Kartusche 50 und verbindet dadurch die Innenseiten der Kartusche 50 und des Behälters 224.

**Fig. 17** zeigt einen Längsschnitt, der senkrecht zur Ebene der Fig. 16 verläuft.

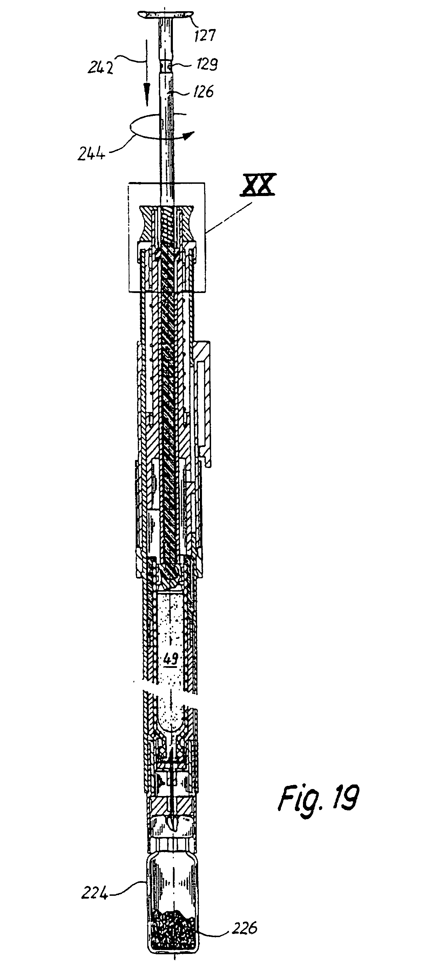

**Fig. 18** zeigt einen vergrößerten Ausschnitt XVIII aus Fig. 17. Man erkennt, dass sich das Gerät 40 in derselben Stellung befindet wie in Fig. 4 und 5.

**Fig. 19** zeigt das Einschrauben der Verlängerungsstange 126 durch Ansetzen in Richtung eines proximalen Pfeiles 242 und Drehen in Richtung eines Drehpfeils 244. Der Rest stimmt mit Fig. 16 überein.

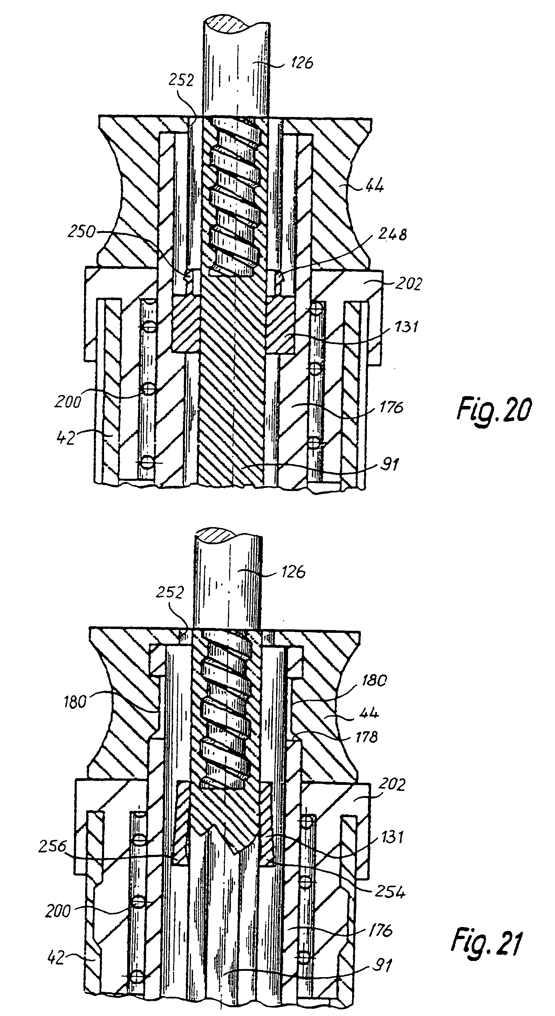

**Fig. 20** zeigt eine vergrößerte Darstellung des Ausschnitts XX der Fig. 19, und Fig. 21 denselben Ausschnitt, aber in einem Schnitt, der senkrecht zur Schnittebene der Fig. 20 verläuft.

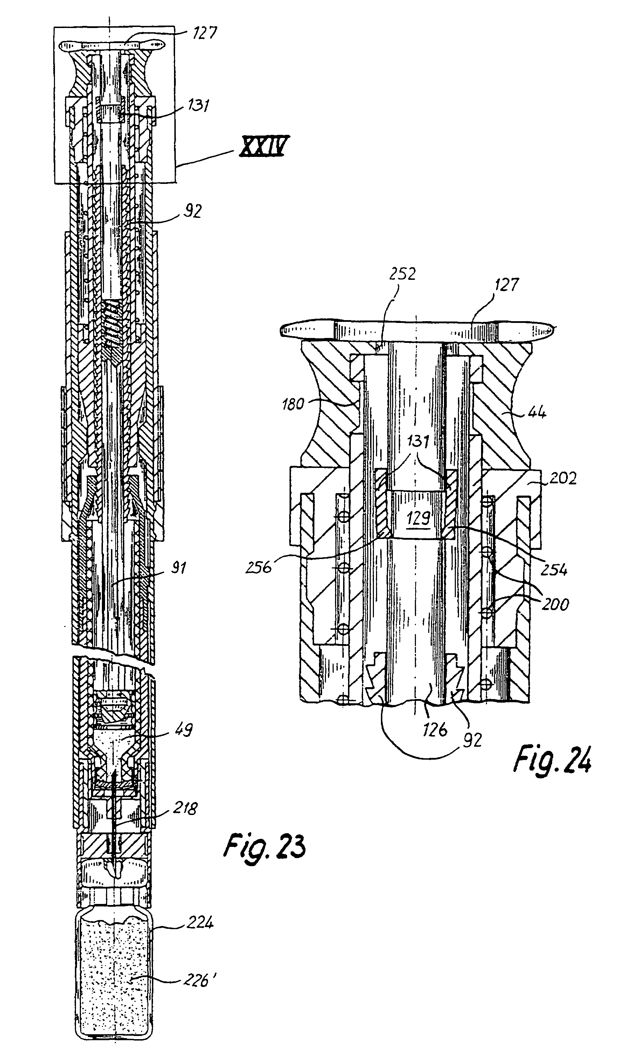

Man erkennt das Signalglied 131, das auf dem Betätigungsglied 91 axial verschiebbar angeordnet ist. Gemäß Fig. 20 und 39 hat es an seinem distalen Ende zwei Widerhaken 248, 250, die so ausgebildet sind, dass sie in einer oberen Öffnung 252 des Betätigungsknopfs 44 einrasten können, wie das z.B. Fig. 3 und 33 zeigen. Ferner hat das Signalglied 131 zwei federnde Abschnitte 254, 256 (Fig. 21), deren proximale Enden so ausgebildet sind, dass sie lösbar in die relativ flache Ringnut 129 der Verlängerungsstange 126 einrasten können, wie das Fig. 24 zeigt.

Gemäß **Fig. 22** wird nun die Verlängerungsstange 126 in Richtung des Pfeiles 242 in proximaler Richtung in das Injektionsgerät 40 gepresst, wodurch der Kolben 90 entsprechend verschoben wird und einen Großteil der Flüssigkeit 49 durch die Nadel 218 in den Behälter 224 presst, wo diese Flüssigkeit 49 das Lyophilisat 226 zu einer Injektionslösung 226' auflöst.

Wie die **Fig. 23** und **24** klar zeigen, ändert bei diesem Vorgang die Zahnstange 92 ihre Lage nicht. Die in medialer Richtung federnd beaufschlagten Teile 254, 256 des Signalglieds 131 rasten mit ihren proximalen Enden lösbar in die Ringnut 129 ein.

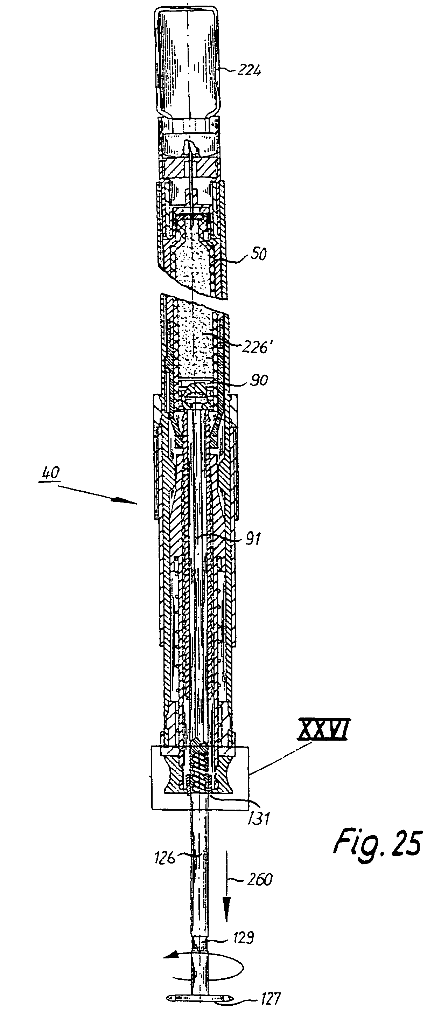

Gemäß **Fig. 25** wird nun das Injektionsgerät 40 auf den Kopf gestellt, und durch Ziehen am Betätigungsglied 127 wird die Verlängerungsstange 126 in Richtung eines Pfeiles 260 in distaler Richtung aus dem Injektionsgerät 40 herausgezogen. Dabei zieht das Betätigungsglied 91 den mit ihm verbundenen Kolben 90 mit sich und saugt die rekonstituierte Flüssigkeit 226' zurück in die Kartusche 50, wo sich diese Flüssigkeit 226' mit den Resten der Flüssigkeit 49 vermischt.

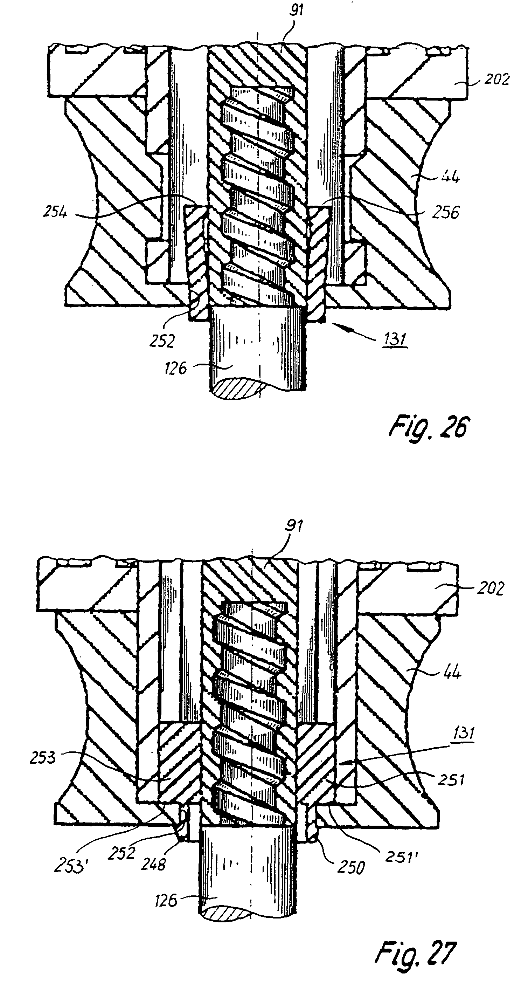

Da das Signalglied 131 hierbei gemäß **Fig. 26 und 27** mit seinen Vorsprüngen 251, 253 am Betätigungsknopf 44 anstößt, kann es der distalen Bewegung der Verlängerungsstange 126 nur auf einem Teil des Weges folgen, d.h. es wird anfangs durch die Ringnut 129 mitgenommen und verrastet schließlich in der Öffnung 252 des Betätigungsknopfes 44. Die Teile 248, 250 haben eine geeignete Signalfarbe und zeigen durch ihre Anwesenheit und Sichtbarkeit an, dass das Injektionsgerät 40 rekonstituierte Injektionsflüssigkeit 226' enthält und gebrauchsfertig ist.

Gemäß **Fig. 28** wird nun der leere Behälter 224 zusammen mit dem Adapter 210 vom Injektionsgerät 40 abgeschraubt (Drehpfeil 264) und abgenommen (Pfeil 266).

Anschließend wird gemäß **Fig. 29** eine Injektionsnadel (Kanüle) 268 mittels ihres Kanülenträgers 270 auf das proximale Ende des Kartuschenhalters 74 aufgeschraubt, wobei ihr distales Ende die Gummimembran 88 der Kartusche 50 durchdringt. Danach wird die Injektionsdosis durch Drehen des Dosierrades 52 eingestellt (Drehpfeil 272), wie bereits bei Fig. 1 und 2 beschrieben, wodurch sich das Dosiseinstellteil 58 mit seinem Clip 62 und der Rastöffnung 66 entsprechend verschiebt, was durch den Pfeil 274 symbolisiert wird. Ein Vergleich der Fig. 1 und 2 zeigt anschaulich, was beim Einstellvorgang vor sich geht.

Zu beachten ist, dass in der Lage gemäß Fig. 29, also vor dem Spannen des Injektionsgeräts 40, die Nadel 268 unten aus dem Gehäuse 42 herausragt. Diese Lage nimmt das Gerät auch nach einer Injektion ein. Fig. 29 zeigt auch eine Nadelschutzkappe 276, welche zur sterilen Montage der Nadel 268 verwendet wird und welche in der Lage gemäß Fig. 29 auf der Nadel 268 belassen werden kann, um diese, und den Benutzer, zu schützen.

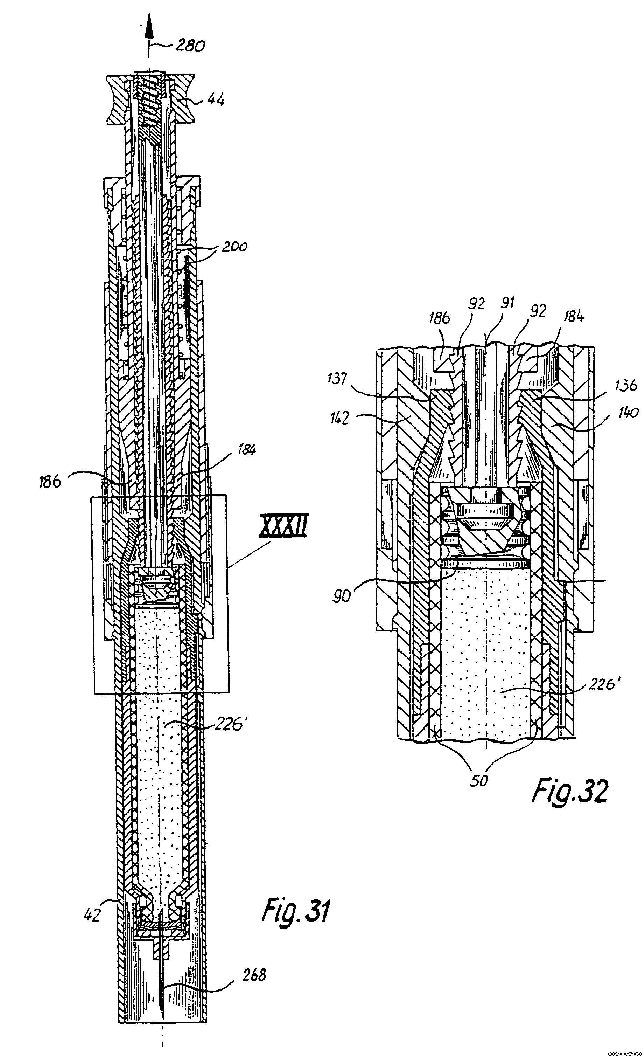

Die **Fig. 30, 31 und 32** zeigen den ersten Teil des Spannvorgangs, mit dem das Injektionsgerät 40 für eine nachfolgende Injektion vorbereitet wird. Der Benutzer zieht hierzu in Richtung eines Pfeiles 280 (Fig. 30, 31) in distaler Richtung am Knopf 44, wodurch die Nadel 268 in das Gehäuse 42 gezogen wird, so dass der Benutzer sie nicht mehr sieht (hidden needle injector). Wie Fig. 30 zeigt, erreicht hierbei das am Stellglied 105 vorgesehene elastische Rastglied 53 noch nicht die Rastöffnung 66, und die Feder 200 wird deshalb nur teilweise gespannt.

Wie die vergrößerte Darstellung der Fig. 32 zeigt, bewegen sich hierbei die Spannbacken 136, 137, welche mit ihren Zähnen 138, 139 (Fig. 5) unter elastischer Vorspannung gegen die Verzahnung der Zahnstange 92 anliegen, zwischen die Vorsprünge 140, 142 im Inneren des Gehäuses 42, und die Spannbacken 184, 186 des Stellglieds 105 gleiten aus der Lage zwischen diesen Vorsprüngen 140, 142 heraus. Es handelt sich hier also um eine Steuerung durch die im Gehäuse 42 vorgesehenen Nocken 140, 142, die man auch als Kulissensteuerung bezeichnen kann.

In der Lage gemäß Fig. 31 und 32 sind sowohl die Spannbacken 136, 137 wie auch die Zahnstange 92 an einer weiteren Bewegung in distaler Richtung gehindert, weil die Spannbacken 136, 137 gegen die Zahnstange 92 gepresst werden und wegen der Verzahnungen mit dieser quasi eine Einheit bilden, und weil die Vorsprünge 140, 142 einen axialen Anschlag für die Spannbacken 136, 137 bilden.

Andererseits können die oberen Spannbacken 184, 186 nun entgegen ihrer in medialer Richtung, also gegen die Zahnstange 92, wirkenden Federkraft federnd nach außen ausweichen, und dies zeigen die Fig. 33 bis 35.

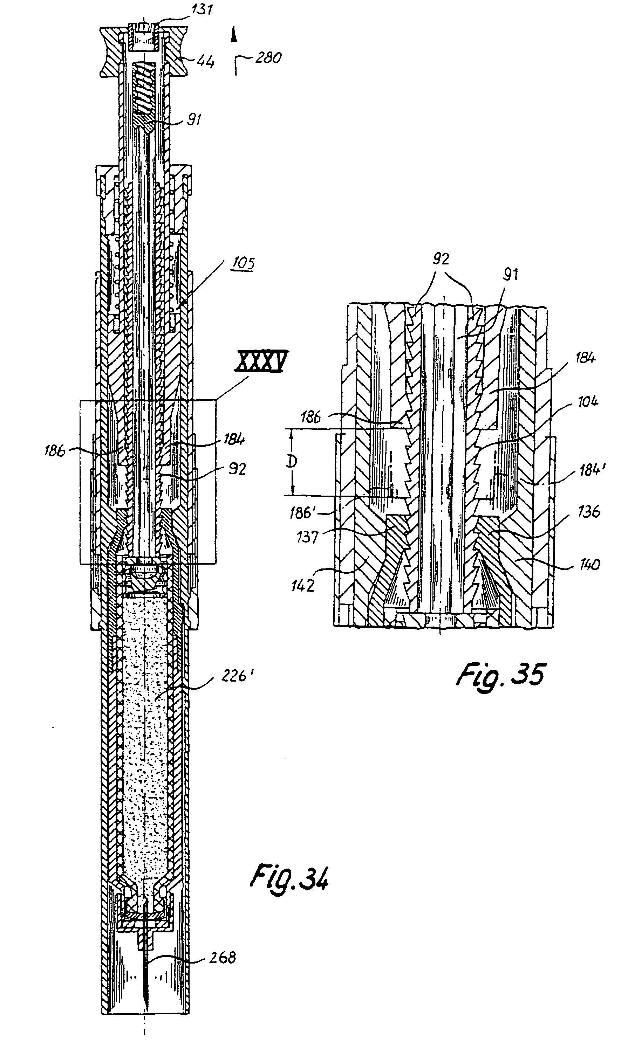

Die **Fig. 33, 34 und 35** zeigen den Abschluss des Bewegungsvorgangs in Richtung des Pfeiles 280, also das vollständige Spannen des Injektionsgeräts 40. Hierbei rastet das elastische Rastglied 53 (am Stellglied 105) in die Ausnehmung 66 des Dosiseinstellteils 58 ein. Bei diesem Vorgang gleiten die Spannbacken 184, 186 des Stellglieds 105 - entsprechend der eingestellten Dosis - über eine entsprechende Zahl von Zähnen 104. Wenn man Fig. 35 mit Fig. 32 vergleicht, erkennt man, dass bei diesem Beispiel die Spannbacken 184, 186 um einen Abstand D von vier Zähnen in distaler Richtung über die Zahnstange 92 geglitten sind. Zur besseren Veranschaulichung ist in Fig. 35 bei 184', 186' die Stellung der Spannbacken gemäß Fig. 32 angedeutet. Bei dieser Bewegung der Spannbacken 184, 186 ist die Zahnstange 92 durch die Spannbacken 136, 137 arretiert, wie bereits bei Fig. 32 beschrieben. Der Abstand D bestimmt die Dosis, die anschließend injiziert wird.

Bei diesem Vorgang entsteht also eine Relativverschiebung zwischen dem Betätigungsknopf 44 und dem distalen Ende des Betätigungsglieds 91, das sich zusammen mit der Zahnstange 92 verschiebt, und man erkennt das in Fig. 34 am oberen Ende.

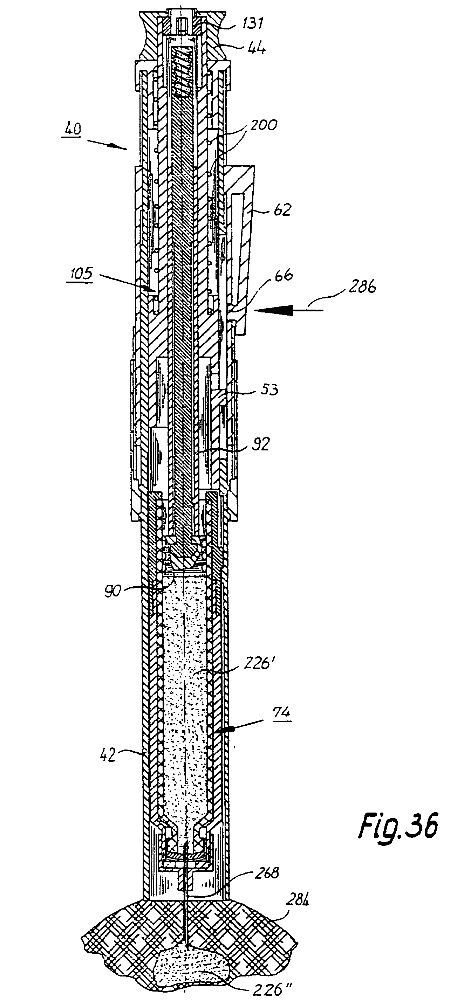

Das Injektionsgerät 40 ist nun gespannt, die richtige Dosis ist eingestellt, und das Gerät ist injektionsbereit. Gemäß Fig. 36 setzt der Patient das Injektionsgerät 40 mit dessen proximalem Ende auf den Körperteil 284 auf, in den injiziert werden soll, drückt in medialer Richtung (Pfeil 286 der Fig. 36) auf den Clip 62, und drückt dadurch das elastische Rastglied 53 aus der Rastöffnung 66. Die gespannte Injektionsfeder 200 drückt dann das Stellglied in proximaler Richtung, und durch den Eingriff der oberen Spannbacken 184, 186 in die Zähne 104 der Zahnstange 92 wird die Bewegung des Stellglieds 105 in vollem Umfang auf die Zahnstange 92 übertragen.

Da am Anfang dieser proximalen Bewegung die unteren Spannbacken 136, 137 durch die Gehäusenocken 140, 142 in Anlage gegen die Zahnstange 92 gehalten werden, wird der Beginn dieser Bewegung durch direkte mechanische Kopplung auf die Nadel 268 übertragen, so dass diese in den Körperteil 284 einsticht.

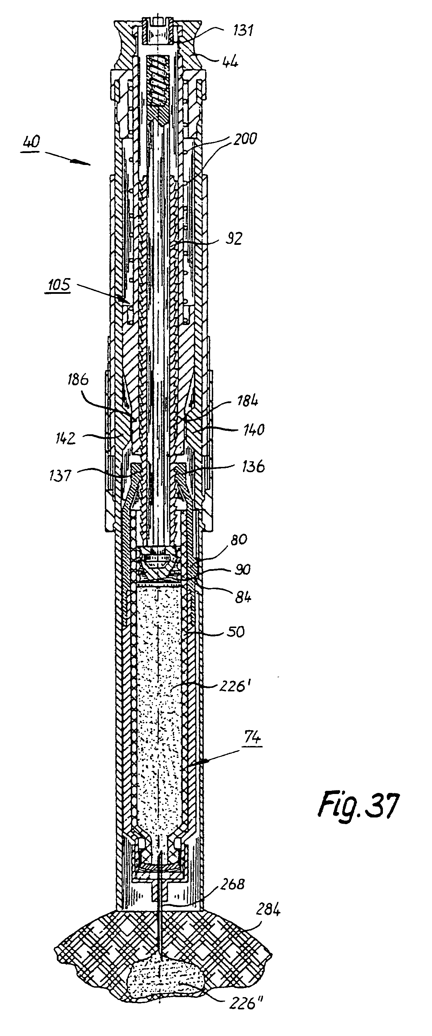

Dadurch gleiten die Spannbacken 136, 137 aus den Gehäusenocken 140, 142 heraus, so dass sie entgegen ihrer Federkraft radial nach außen ausweichen können, wodurch die Zahnstange 92 nun beginnt, den Kolben 90 in der Kartusche 50 in proximaler Richtung zu verschieben. Dadurch steigt der Druck in der Injektionsflüssigkeit 226' an, und dieser erhöhte hydraulische Druck bewirkt eine Fortsetzung der proximalen Bewegung der - bereits eingestochenen - Nadel 268. Dabei gelangen die radialen Vorsprünge 80 des Kartuschenhalters 74 in Anlage gegen den Anschlag 84, wodurch die Bewegung des Kartuschenhalters 74 gestoppt wird. Durch die restliche Bewegung der Zahnstange 92 - um den Weg D (Fig. 35) - wird die zuvor eingestellte Flüssigkeitsmenge aus der Kartusche 50 in den Körperteil 284 gepresst, wo diese Flüssigkeit in Fig. 36 und 37 mit 226" bezeichnet ist.

Wenn die Dosiseinstellung unverändert bleibt, muss der Benutzer anschließend nur, ggf. nach Auswechseln der Injektionsnadel 268, mit dem Spannknopf 44 das Injektionsgerät 40 wieder in die Stellung gemäß Fig. 33 bringen und kann dann erneut eine Injektion durchführen. Nach der Rekonstitution ist also die Benutzung des Injektionsgeräts 40 denkbar einfach und bereitet auch einem sehschwachen Patienten keine Schwierigkeiten.

## Patentansprüche

1. Injektionsgerät,
mit einem Gehäuse (42) zur Aufnahme eines Behälters (50) mit einer Flüssigkeit (49; 226'),
in welchem Behälter (50) ein Kolben (90) verschiebbar angeordnet ist, um durch Druck auf das distale Ende dieses Kolbens (90) Flüssigkeit aus dem Behälter (50) auspressen zu können,
mit einem in dem Gehäuse (42) verschiebbar angeordneten Stößel (92), welcher zur Anlage gegen das distale Ende des Kolbens (90) ausgebildet ist und die Übertragung einer Kraft auf den Kolben (90) in proximaler Richtung ermöglicht, **dadurch gekennzeichnet, dass** es ein
mit einem dem Stößel (92) zugeordneten und relativ zu diesem verschiebbares Glied (91) aufweist, welches an seinem proximalen Endbereich mit dem Kolben (90) so verbindbar ist, dass es die Übertragung einer Kraft in proximaler Richtung und die Übertragung einer Kraft in distaler Richtung auf den Kolben (90) ermöglicht.

2. Injektionsgerät nach Anspruch 1, bei welchem das verschiebbare Glied (91) an seinem distalen Endbereich mit einer Kupplungsvorrichtung (124) versehen ist.

3. Injektionsgerät nach Anspruch 2, bei welchem die Kupplungsvorrichtung (124) zur lösbaren Verbindung mit einem Verlängerungsglied (126) dient, welches dazu ausgebildet ist, das verschiebbare Glied (91) zu verlängern,

4. Injektionsgerät nach einem oder mehreren der vorhergehenden Ansprüche, bei welchem das verschiebbare Glied (91) in einer Ausnehmung (114) des Stößels (92) verschiebbar angeordnet ist.

5. Injektionsgerät nach Anspruch 4, bei welchem das verschiebbare Glied (91) in der Ausnehmung (114) des Stößels (92) unverdrehbar geführt ist.

6. Injektionsgerät nach Anspruch 4 oder 5, bei welchem dem verschiebbaren Glied (91) mindestens ein Anschlag (118, 119) zugeordnet ist, um seine axiale Bewegung relativ zum Stößel (92) zu begrenzen.

7. Injektionsgerät nach einem der vorhergehenden Ansprüche, bei welchem der Stößel als Zahnstange (92) ausgebildet ist.

8. Injektionsgerät nach Anspruch 7, bei welchem ein Stellglied (105) vorgesehen ist, welches mit mindestens einem federnden Rastzahn (184', 186') zum federnden Eingriff mit den Zähnen (104) der Zahnstange (92) versehen ist.

9. Injektionsgerät nach Anspruch 8, bei welchem dem mindestens einen federnden Rastzahn (184', 186') des Stellglieds (105) eine Nockensteuerung (140, 142) zugeordnet ist, welche, gesteuert von der axialen Lage des Stellglieds (105) relativ zum Gehäuse (42), eine federnde Auslenkung dieses mindestens einen Rastzahns (184', 186') blockiert.

10. Injektionsgerät nach einem der Ansprüche 7 bis 9, bei welchem der Behälter (50) relativ zum Gehäuse (42) axial verschiebbar ist, um durch Verschieben des Behälters (50) eine Einstichbewegung einer mit dem Behälter (50) verbundenen Kanüle (268) in die Haut des Patienten zu ermöglichen.

11. Injektionsgerät nach Anspruch 10, bei welchem mit dem verschiebbaren Behälter (50) mindestens ein federnder Rastzahn (138, 139) verbunden sind, welcher elastisch gegen Zähne (104) der Zahnstange (92) anliegt.

12. Injektionsgerät nach Anspruch 11, bei welchem dem mindestens einen federnden Rastzahn (138, 139) des Behälters (50) eine Nockensteuerung (140, 142) zugeordnet ist, welche, gesteuert von der axialen Lage des Behälters (50) relativ zum Gehäuse (42), eine federnde Auslenkung dieses mindestens einen Rastzahns (138, 139) blockiert.

13. Injektionsgerät nach einem der Ansprüche 7 bis 12, bei welchem zwischen dem verschiebbaren Behälter (50) und der Zahnstange (92) eine von der axialen Stellung des Behälters (50) relativ zum Gehäuse (42) beeinflusste Verbindung (136, 137) vorgesehen ist.

14. Injektionsgerät nach einem der vorhergehenden Ansprüche, bei welchem ein Signalglied (131) vorgesehen ist, das durch den Rekonstitutionsvorgang betätigbar ist.

15. Injektionsgerät nach einem oder mehreren der vorhergehenden Ansprüche, mit einer Feder (200) zum Speichern von Energie für eine Injektion, welche Feder (200) durch Verschieben eines Stellglieds (105) in distaler Richtung spannbar ist,
und mit einem Rastglied (53, 66) zum lösbaren Verrasten des Stellglieds (105) in dieser verschobenen Stellung.

16. Injektionsgerät nach Anspruch 15, bei welchem im Gehäuse (42) mindestens ein Nocken (140, 142) vorgesehen ist, welcher in der verschobenen Stellung des Stellglieds (105) eine lageabhängige mechanische Verbindung (136, 137) zwischen Zahnstange (92) und Behälter (50) aktiviert.

17. Injektionsgerät nach einem oder mehreren der vorhergehenden Ansprüche, bei welchem dem Behälter (50) ein Halter (74) zugeordnet ist, welcher im Gehäuse (42) axial zwischen einer proximalen und einer distalen Endstellung verschiebbar ist und zur Aufnahme des Behälters (50) ausgebildet ist.

18. Injektionsgerät nach Anspruch 17, bei welchem die Länge des Halters (74) veränderbar ist.

19. Injektionsgerät nach Anspruch 18, bei welchem der Halter (74) einen proximalen Abschnitt (76) und einen distalen Abschnitt (78) aufweist, welche durch eine verstellbare Verbindung (77) miteinander verbunden sind, die eine Änderung der Gesamtlänge des Halters (74) ermöglicht.

20. Injektionsgerät nach Anspruch 19, bei welchem die verstellbare Verbindung eine Mikrorastung (77) aufweist, welche einen proximalen Abschnitt (76) und einen distalen Abschnitt (78) des Halters (74) in verstellbarer Weise miteinander verbindet.

21. Injektionsgerät nach einem oder mehreren der Ansprüche 17 bis 20, bei welchem der Halter (74) in seinem distalen Bereich mit Anschlagmitteln (80) versehen ist, welche seine proximale und/oder seine distale Endstellung relativ zum Gehäuse (42) festlegen, und bei welchem die Längenverstellung des Halters (74) relativ zu diesen Anschlagmitteln erfolgt, so dass bei einer Längenänderung des Halters (74) die Lage des Behälters (50) relativ zur Zahnstange (92) verändert wird.

22. Injektionsgerät nach einem oder mehreren der vorhergehenden Ansprüche, bei welchem ein relativ zum Gehäuse (42) in Längsrichtung verschiebbares Dosiseinstellteil (58) Rastmittel (66) zur Verrastung eines Rastorgans (53) aufweist.

23. Injektionsgerät nach Anspruch 22, bei welchem am verschiebbaren Dosiseinstellteil (58) ein Glied (62) zum Lösen der Rastung zwischen den Rastmitteln (66) und dem Rastorgan (53) vorgesehen ist.

24. Injektionsgerät nach Anspruch 22 oder 23, bei weichem am Gehäuse (42) eine relativ zu diesem verdrehbare aber relativ zum ihm nicht axial verschiebbare Gewindehülse (52) vorgesehen ist, welche mit einem Gewinde (56) am verschiebbaren Dosiseinstellteil (58) in Eingriff steht.

25. Injektionsgerät nach einem oder mehreren der Ansprüche 22 bis 24, bei welchem das verschiebbare Dosiseinstellteil (58) relativ zum Gehäuse (42) nicht verdrehbar ist.

26. Verfahren zur Rekonstitution einer Injektionsflüssigkeit in einem Injektionsgerät zur nachfolgenden Verwendung der rekonstituierten Injektionsflüssigkeit in diesem Gerät,
welches Injektionsgerät
- ein Gehäuse zur Aufnahme einer mit einem Kolben versehenen Kartusche für eine Flüssigkeit,
- einen Stößel zur Beaufschlagung dieses Kolbens bei einer Injektion,
- und ein relativ zu diesem Stößel verschiebbares und mit dem Kolben verbindbares Glied aufweist,
mit folgenden Schritten:
a) Mit der Kartusche wird ein Behälter in Flüssigkeitsverbindung gebracht, der eine Komponente für die Rekonstitution der Injektionsflüssigkeit enthält;
b) durch Verschieben des verschiebbaren Glieds in einer vorgegebenen Richtung wird Flüssigkeit aus der Kartusche in diesen Behälter gepumpt, um dort die Injektionsflüssigkeit zu rekonstituieren;
c) durch Verschieben des verschiebbaren Glieds in einer zur vorgegebenen Richtung entgegengesetzten Richtung wird die rekonstituierte Flüssigkeit aus dem Behälter in die Kartusche zurückgesaugt;
d) der Behälter wird entfernt.

27. Verfahren nach Anspruch 26, bei welchem das verschiebbare Glied beim Schritt c) in der zur vorgegebenen Richtung entgegengesetzten Richtung so weit verschoben wird, bis der Kolben zur Anlage gegen den Stößel kommt.

28. Verfahren nach Anspruch 26 oder 27, bei welchem durch die Wirkung von in einer Feder gespeicherter Energie eine mit der Kartusche verbundene Injektionskanüle eingestochen und nach dem Einstich Injektionsflüssigkeit injiziert wird,
wobei der Einstich der Injektionskanüle mittels der gespeicherten Energie zuerst durch mechanischen Direktantrieb der Injektionskanüle und anschließend durch hydraulische Kraftübertragung mittels Druckerhöhung in der Kartusche erfolgt.

## Claims

1. Injection device comprising
a housing (42) for receiving a container (50) with a liquid (49; 226'),
which container (50) has a piston (90) arranged displaceably therein in order to be able to express liquid out of the container (50) through pressure on the distal end of this piston (50),
a push rod (92) displaceably arranged in the housing (42) and designed to bear against the distal end of the piston (90) and permitting transmission of a force on the piston (90) in the proximal direction,
**characterised in that** the injection device comprises a member (91) which is associated with the push rod (92) and displaceable relative to the latter and which in its proximal end area can be connected to the piston (90) in such a way that it allows the transmission of a force in the proximal direction and the transmission of a force in the distal direction to the piston (90).

2. Injection device according to claim 1, in which the displaceable member (91) is provided with a coupling device (124) in its distal end area.

3. Injection device according to claim 2, in which the coupling device (124) serves for releasable connection to an extension member (126) which is designed to lengthen the displaceable member (91).

4. Injection device according to one or more of the preceding claims, in which the displaceable member (91) is arranged displaceably in a recess (114) of the push rod (92).

5. Injection device according to claim 4, in which the displaceable member (91) is guided so that it cannot turn in the recess (114) of the push rod (92).

6. Injection device according to claim 4 or 5, in which the displaceable member (91) has associated therewith at least one abutment (118, 119) in order to limit its axial movement relative to the push rod (92).

7. Injection device according to one of the preceding claims, in which the push rod is embodied as a toothed rod (92).

8. Injection device according to claim 7, in which a control member (105) is provided which is provided with at least one sprung detent tooth (184', 186') for resilient engagement with the teeth (104) of the toothed rod (92).

9. Injection device according to claim 8, in which the at least one sprung detent tooth (184', 186') of the control member (105) has associated therewith a cam control (140, 142) which, controlled by the axial position of the control member (105) relative to the housing (42), prevents this at least one detent tooth (184', 186') from yielding resiliently.

10. Injection device according to one of claims 7 to 9, in which the container (50) is displaceable axially relative to the housing (42) in order to allow an insertion movement of a cannula (268) connected to the container (50) into the skin of the patient through displacement of the container (50).

11. Injection device according to claim 10, in which the displaceable container (50) is connected to at least one sprung detent tooth (138, 139) which bears elastically against teeth (104) of the toothed rod (92).

12. Injection device according to claim 11, in which the at least one sprung detent tooth (138, 139) of the container (50) has associated therewith a cam control (140, 142) which, controlled by the axial position of the container (50) relative to the housing (42), prevents this at least one detent tooth (138, 139) from yielding resiliently.

13. Injection device according to one of claims 7 to 12, in which a connection (136, 137) influenced by the axial position of the container (50) relative to the housing (42) is provided between the displaceable container (50) and the toothed rod (92).

14. Injection device according to one of the preceding claims, in which a signalling member (131) is provided which can be operated by the reconstitution operation.

15. Injection device according to one or more of the preceding claims, comprising a spring (200) for storage of energy for an injection, which spring (200) can be cocked by displacement of a control member (105) in the distal direction,
and comprising a detent member (53, 66) for releasable locking of the control member (105) in this displaced position.

16. Injection device according to claim 15, in which at least one cam (140, 142) is provided in the housing (42), which cam activates a position-dependent mechanical connection (136, 137) between the toothed rod (92) and the container (50) in the displaced position of the control member (105).

17. Injection device according to one or more of the preceding claims, in which the container (50) has associated therewith a holder (74) which is displaceable axially in the housing (42) between a proximal and a distal end position and is designed for reception of the container (50).

18. Injection device according to claim 17, in which the length of the holder (74) is variable.

19. Injection device according to claim 18, in which the holder (74) comprises a proximal portion (76) and a distal portion (78), which are connected to one another by an adjustable connection (77) which allows alteration of the overall length of the holder (74).

20. Injection device according to claim 19, in which the adjustable connection comprises a microdetent (77) which connects a proximal portion (76) and a distal portion (78) of the holder (74) to one another in an adjustable manner.

21. Injection device according to one or more of claims 17 to 20, in which the holder (74) is provided in its distal area with abutment means (80) which determine its proximal and/or distal end position relative to the housing (42),
and in which the adjustment of the length of the holder (74) is carried out relative to these stop means so that when the length of the holder (74) is altered, the position of the container (50) relative to the toothed rod (92) is changed.

22. Injection device according to one or more of the preceding claims, in which a dose-setting part (58) displaceable relative to the housing (42) in the longitudinal direction comprises detent means (66) for locking a detent member (53).

23. Injection device according to claim 22, in which a member (62) is provided on the displaceable dose-setting part (58) for releasing the detent connection between the detent means (66) and the detent member (53).

24. Injection device according to claim 22 or 23, in which the housing (42) is provided with a threaded sleeve (52) which is rotatable relative to the housing but not displaceable axially relative thereto and which is in engagement with a thread (56) on the displaceable dose-setting part (58).

25. Injection device according to one or more of claims 22 to 24, in which the displaceable dose-setting part (58) is not rotatable relative to the housing (42).

26. Method for reconstituting an injection liquid in an injection device for subsequent use of the reconstituted injection liquid in this device,
said injection device comprising
- a housing for receiving a cartridge with a piston for a liquid,
- a push rod for operating this piston during an injection,
- and a member which is displaceable relative to this push rod and can be connected to the piston,
- comprising the following steps:
a) a container which contains a component for reconstituting the injection liquid is brought into liquid connection with the cartridge;
b) liquid is pumped out the cartridge into this container by displacement of the displaceable member in a predetermined direction in order to reconstitute the injection liquid in it;
c) the reconstituted liquid is sucked back out of the container into the cartridge by displacement of the displaceable member in the opposite direction to the predetermined direction;
d) the container is removed.

27. Method according to claim 26, in which the displaceable member is displaced in the opposite direction to the predetermined direction in step c) until the piston comes to bear against the push rod.

28. Method according to claim 26 or 27, in which an injection cannula connected to the cartridge is inserted and after insertion injection fluid is injected through the effect of energy stored in a spring, during which the insertion of the injection cannula by means of the stored energy takes place first through mechanical direct drive of the injection cannula and then through hydraulic transmission of force by means of increasing the pressure in the cartridge.

## Revendications

1. Appareil pour injection comportant un boîtier (42) destiné à contenir un réservoir (50) renfermant un liquide (49 ; 226'), réservoir (50) dans lequel un piston (90) est disposé de façon à être mobile, pour pouvoir expulser du liquide hors du réservoir (50) par le biais de la pression exercée sur l'extrémité distale de ce piston (90), comportant aussi une tige-poussoir (92) disposée de façon à être mobile dans le boîtier (42), qui est conçue pour s'appuyer contre l'extrémité distale du piston (90) et qui permet de transmettre une force sur le piston (90) dans le sens proximal, **caractérisé en ce que** l'appareil pour injection (40) comporte un élément (91) associé à la tige-poussoir (92) et mobile par rapport à celle-ci, qui, dans sa zone d'extrémité proximale peut être relié au piston (90) de sorte qu'il permet la transmission d'une force au piston (90) dans le sens distal et dans le sens proximal.

2. Appareil pour injection selon la revendication 1, dans lequel l'élément déplaçable (91) est pourvu dans sa zone d'extrémité distale d'un dispositif d'accouplement (124).

3. Appareil pour injection selon la revendication 2, dans lequel le dispositif d'accouplement (124) sert au raccordement détachable à un élément de rallonge (126), lequel est conçu pour prolonger l'élément déplaçable (91).

4. Appareil pour injection selon une ou plusieurs des revendications précédentes, dans lequel l'élément déplaçable (91) est disposé dans un creux ou évidement (114) de la tige-poussoir (92) de façon mobile.

5. Appareil pour injection selon la revendication 4, dans lequel l'élément déplaçable (91) est guidé dans le creux (114) de la tige-poussoir (92) de façon à ne pas pouvoir y pivoter.

6. Appareil pour injection selon l'une des revendications 4 ou 5, dans lequel au moins une butée (118, 119) est associée à l'élément déplaçable (91) de façon à limiter son déplacement axial par rapport à la tige-poussoir (92).

7. Appareil pour injection selon l'une des revendications précédentes, dans lequel la tige-poussoir (92) est conçue sous la forme d'une crémaillère.

8. Appareil pour injection selon la revendication 7, dans lequel un élément de réglage (105) est prévu, qui est pourvu d'au moins une dent à encliqueter élastique (184', 186') destinée à se prendre ou à s'engager élastiquement dans les dents (104) de la crémaillère (92).

9. Appareil pour injection selon la revendication 8, dans lequel une commande par cames (140, 142) est associée à ladite au moins une dent à encliqueter élastique (184', 186') de l'élément de réglage (105), qui, commandée par la position axiale de l'élément de réglage (105) par rapport au boîtier (42), bloque la déviation élastique de cette dite au moins une dent à encliqueter (184', 186').

10. Appareil pour injection selon l'une des revendications 7 à 9, dans lequel le réservoir (50) est déplaçable axialement par rapport au boîtier (42), de façon à permettre, par déplacement du réservoir (50), un mouvement de piqûre dans la peau d'un patient d'une canule (268) reliée au réservoir (50).

11. Appareil pour injection selon la revendication 10, dans lequel au moins une dent à encliqueter élastique (138, 139) est reliée au réservoir déplaçable (50), qui s'appuie de façon élastique contre des dents (104) de la crémaillère (92).

12. Appareil pour injection selon la revendication 11, dans lequel une commande par cames ou commande à came (140, 142) est associée à ladite au moins une dent à encliqueter élastique (138, 139) du réservoir (50), commande qui, commandée par la position axiale du réservoir (50) par rapport au boîtier (42), bloque une déviation élastique de cette dite au moins une dent à encliqueter (138, 139).

13. Appareil pour injection selon l'une des revendications 7 à 12, dans lequel un raccord (136, 137) influencé par la position axiale du réservoir (50) par rapport au boîtier (42) est prévu entre le réservoir déplaçable (50) et la crémaillère (92).

14. Appareil pour injection selon l'une des revendications précédentes, dans lequel un élément indicateur (131) est prévu, qui peut être actionné par le processus de reconstitution.

15. Appareil pour injection selon une ou plusieurs des revendications précédentes, comportant un ressort (200) pour accumuler de l'énergie en vue d'une injection, lequel ressort à injection (200) pouvant être mis sous tension par déplacement d'un élément de réglage (105) dans le sens distal, et comportant un élément à encliqueter (53, 66) pour le verrouillage amovible de l'élément de réglage (105) dans cette position déplacée.

16. Appareil pour injection selon la revendication 15, dans lequel au moins une came (140, 142) est prévue dans le boîtier (42), qui, en position déplacée de l'élément de réglage (105), actionne, entre la crémaillère (92) et le réservoir (50), un raccord mécanique (136, 137), qui dépend du positionnement.

17. Appareil pour injection selon une ou plusieurs des revendications précédentes, dans lequel un support (74) est associé au réservoir (50), qui est axialement déplaçable dans le boîtier (42) entre une position d'extrémité proximale et une position d'extrémité distale et qui est conçu pour accueillir le réservoir (50).

18. Appareil pour injection selon la revendication 17, dans lequel la longueur du support (74) est modifiable.

19. Appareil pour injection selon la revendication 18, dans lequel le support (74) comporte un segment proximal (76) et un segment distal (78), qui sont reliés entre eux par l'intermédiaire d'un raccord ajustable (77) qui permet de modifier la longueur globale du support (74).

20. Appareil pour injection selon la revendication 19, dans lequel le raccord ajustable comprend un microdispositif à encliqueter (77) qui relie entre eux de façon ajustable un segment proximal (76) et un segment distal (78) du support (74).

21. Appareil pour injection selon une ou plusieurs des revendications 17 à 20, dans lequel le support (74) est pourvu dans sa zone d'extrémité distale d'éléments de butée (80), qui fixent sa position d'extrémité proximale et/ou distale par rapport au boîtier (42), et dans lequel l'ajustement de la longueur du support (74) est réalisé par rapport à ces éléments de butée de sorte que lorsqu'il y a modification de la longueur du support (74), la position du réservoir (50) par rapport à la crémaillère (92) est modifiée.

22. Appareil pour injection selon une ou plusieurs des revendications précédentes, dans lequel un élément doseur (58) déplaçable dans le sens longitudinal par rapport au boîtier (42) est pourvu d'éléments à encliqueter (66) pour l'encliquetage d'un élément à encliqueter (53).

23. Appareil pour injection selon la revendication 22, dans lequel un élément (62) pour défaire l'encliquetage entre les éléments à encliqueter (66) et l'élément à encliqueter (53) est prévu au niveau de l'élément doseur déplaçable (58).

24. Appareil pour injection selon la revendication 22 ou 23, dans lequel une douille filetée (52) est prévue au niveau du boîtier (42) et qui peut tourner par rapport au boîtier mais qui ne peut être déplacée axialement par rapport à celui-ci, et qui est engagée dans un filet (56) prévu au niveau de l'élément doseur déplaçable (58).

25. Appareil pour injection selon une ou plusieurs des revendications 22 à 24, dans lequel l'élément doseur déplaçable (58) ne peut tourner par rapport au corps (42).

26. Procédé de reconstitution d'un liquide pour injection dans un appareil pour injection en vue de l'utilisation ultérieure dans cet appareil du liquide pour injection reconstitué, lequel appareil pour injection comportant :
- un boîtier destiné à accueillir une cartouche pour le liquide, dotée d'un piston,
- une tige-poussoir pour l'entraînement de ce piston lors d'une injection,
- et un élément déplaçable par rapport à cette tige-poussoir et pouvant être relié au piston,
lequel procédé comportant les étapes consistant à :
a) établir un raccord hydraulique entre la cartouche et un réservoir contenant un composant pour la reconstitution du liquide pour injection ;
b) pomper le liquide depuis la cartouche dans ce réservoir par déplacement de l'élément déplaçable dans un sens prédéterminé, pour reconstituer, dans le réservoir, le liquide pour injection ;
c) réinjecter dans la cartouche le liquide reconstitué provenant du réservoir, par déplacement de l'élément déplaçable dans le sens opposé au sens prédéterminé ;
d) ôter le réservoir.

27. Procédé selon la revendication 26, dans lequel l'élément déplaçable de l'étape c) est poussé dans le sens opposé au sens prédéterminé jusqu'à ce que le piston vienne s'appuyer contre la tige-poussoir.

28. Procédé selon la revendication 26 ou 27, dans lequel une canule d'injection reliée à la cartouche, sous l'effet de l'énergie accumulée dans un ressort, est percée, et en ce que, après la piqûre, du liquide pour injection est injecté, la percée de la canule d'injection par le biais de l'énergie stockée s'effectuant d'abord par entraînement mécanique direct de la canule d'injection, puis par la transmission par force hydraulique du fait de l'augmentation de la pression dans la cartouche.
